(19) Europäisches Patentamt
European Patent Office
Office européen des brevets

(11) **EP 2 791 676 B1**

(12) **EUROPEAN PATENT SPECIFICATION**

(45) Date of publication and mention
of the grant of the patent:
**05.08.2020 Bulletin 2020/32**

(21) Application number: **12857141.1**

(22) Date of filing: **17.12.2012**

(51) Int Cl.:
**G01N 33/68** *(2006.01)*

(86) International application number:
**PCT/CA2012/001157**

(87) International publication number:
**WO 2013/086624 (20.06.2013 Gazette 2013/25)**

(54) **METHODS FOR DIAGNOSING ALZHEIMER'S DISEASE**

VERFAHREN ZUR DIAGNOSE VON MORBUS ALZHEIMER

MÉTHODES DE DIAGNOSTIC DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2011 US 201161576168 P**
**09.03.2012 CA 2771621**

(43) Date of publication of application:
**22.10.2014 Bulletin 2014/43**

(73) Proprietor: **Telo Genomics Holdings Corp.**
**Toronto, ON M5G 1L7 (CA)**

(72) Inventors:
• **MAI, Sabine**
**Winnipeg,**
**Manitoba R3N 1M9 (CA)**
• **GARCIA, Angeles**
**Kingston,**
**Ontario K7K 5S3 (CA)**

(74) Representative: **Greaves Brewster LLP**
**Copa House**
**Station Road**
**Cheddar, Somerset BS27 3AH (GB)**

(56) References cited:
**WO-A2-99/66075     WO-A2-2010/118419**

• **LUKENS J N ET AL: "Comparisons of telomere lengths in peripheral blood and cerebellum in Alzheimer's disease", ALZHEIMER'S & DEMENTIA: THE JOURNAL OF THE ALZHEIMER'SASSOCIATION, ELSEVIER, NEW YORK, NY, US, vol. 5, no. 6, 1 November 2009 (2009-11-01), pages 463-469, XP026743205, ISSN: 1552-5260, DOI: 10.1016/J.JALZ.2009.05.666 [retrieved on 2009-11-05]**
• **RIUDAVETS ET AL: "Resistance to Alzheimer's pathology is associated with nuclear hypertrophy in neurons", NEUROBIOLOGY OF AGING, TARRYTOWN, NY, US, vol. 28, no. 10, 14 August 2007 (2007-08-14), pages 1484-1492, XP022197105, ISSN: 0197-4580, DOI: 10.1016/J.NEUROBIOLAGING.2007.05.005**
• **D M MANN ET AL: "Alterations in protein synthetic capability of nerve cells in Alzheimer's disease.", JOURNAL OF NEUROLOGY NEUROSURGERY & PSYCHIATRY., vol. 44, no. 2, 1 February 1981 (1981-02-01), pages 97-102, XP55234845, GB ISSN: 0022-3050, DOI: 10.1136/jnnp.44.2.97**
• **MATHUR SHUBHA ET AL: "Three-dimensional quantitative imaging of telomeres in buccal cells identifies mild, moderate, and severe Alzheimer's disease patients", JOURNAL OF ALZHEIMER'S DISEASE : JAD, IOS PRESS, NL, vol. 39, no. 1, 1 January 2014 (2014-01-01), pages 35-48, XP009187627, ISSN: 1875-8908, DOI: 10.3233/JAD-130866**

EP 2 791 676 B1

**(Cont. next page)**

- THOMAS ET AL.: 'Telomere length in white blood cells, buccal cells and brain tissue and its variation with ageing and Alzheimer's disease' MECHANISMS OF AGEING AND DEVELOPMENT vol. 129, no. 4, 2008, pages 183 - 190, XP022507269
- PANOSSIAN ET AL.: 'Telomere shortening in T cells correlates with Alzheimer's disease status' NEUROBIOLOGY OF AGING vol. 24, no. 1, 2003, pages 77 - 84, XP055073085
- JENKINS ET AL.: 'Increased ''absence'' of telomeres may indicate Alzheimer's disease/dementia status in older individuals with Down syndrome' NEUROSCIENCE LETTERS vol. 440, no. 3, 2008, pages 340 - 343, XP022778894
- DE VOS ET AL.: 'Controlled light exposure microscopy reveals dynamic telomere microterritories throughout the cell cycle' CYTOMETRY PART A vol. 75A, no. 5, 2009, pages 428 - 439, XP055073087

## Description

### Field of the Disclosure

The disclosure relates to diagnostic methods for dementias such as Alzheimer's disease and particularly to methods involving characterizing the organization of telomeres to diagnose Alzheimer's disease or an increased risk of developing Alzheimer's disease.

### Background of the Disclosure

The ends of linear chromosomes are capped by telomeres. Human telomeres consist of repetitive two thymidine (TT), one adenine (A) and 3 glycine (GGG) subunits, which are associated with a variety of telomere-binding proteins known as the sheltering complex (Blackburn et al., 1994, de Lange et al., 2002).

Telomeres get progressively shorter with each cell division. This process occurs because the DNA-replication machinery is incapable of fully replicating the ends of linear molecules, and, degradation and oxidative damage of nucleotides in DNA. Telomerase is an enzyme, which has the ability to prevent telomeres from shortening although most of the cells do not express sufficient quantities of this enzyme to prevent this process. As a result, telomeres shorten with age in tissues and cells (Kenkichi et al., 2001, Harley et al., 2001, Huffman et al., 1990).

The function of telomeres is to mask and protect the ends of chromosomes from exposure to DNA damage. Telomeres maintain chromosome integrity. When telomere ends are unprotected, genomic instability is triggered. Genomic instability has been implicated as a major causal factor in cancer and aging (Charames et al., 2003, Holland et al., 2009, Hanialra et al., 2011).

Genomic instability is a crucial step in the development of most cancers. It has been suggested that inactivation of DNA repair pathways, which leads to an increased mutation rate and chromosomal instability, can initiate and accelerate the neoplastic process (Lothe et al., 1993, Rudolph et al., 1999, Colleu-Durel et al., 2001, Chan et al., 2002).

Genomic instability increases with age (Slagboom et al., 1999). There are a few potential mechanisms that have been proposed to explain age-dependent genome instability. These include the accumulation of oxidative damage to DNA, defects in mitochondrial functions that promote oxidative stress and DNA damage, mutations in proteins required for efficient DNA replication, DNA repair and checkpoints, telomere erosion and epigenetic effects on DNA repair and other genome maintenance programs (Hayflick et al., 1977, Sohal et al., 1985, Harley et al., 1990).

Telomeres become shorter during our life. Accumulation of short telomeres in our tissues contributes to pathological conditions such as congenital dyskeratosis, Werner premature aging syndrome and Alzheimer's disease (Yu et al., 1996, Shen et al., 1998, Fry et al., 1999, Burns et al., 2002, Panossian et al., 2003, Thomas et al., 2007).

Studies on telomere lengths in patients with Alzheimer's disease (AD) have revealed contrary results. Telomere shortening in AD seems to be cell type dependent (Panossian et al., 2003, Baird et al., 2004, Thomas et al., 2008). Short telomeres are found in cells such as lymphocytes, leukocytes, peripheral blood mononuclear cells, fibroblast cells, and buccal cells (BCs) from Alzheimer's patients (Jenkit et al., 2003, Panossian et al., 2003, Honig et al., 2006, Lukaset et al., 2009) whereas in brain tissue such as the hippocampus, telomeres have been found to be longer than in controls (Thomas et al., 2008). These findings indicate important differences in telomere maintenance in AD patients in different groups of cells.

AD is a neurodegenerative condition resulting in neuronal death. AD patients show symptoms of impaired memory, judgment and decision-making among other cognitive disabilities (Burns et al., 2002, Du et al., 2001). AD patients are currently diagnosed on clinical grounds while excluding other causes of dementia. The two histopathological structures present within the brain that positively identify AD conclusively at post-mortem are the neurofibrillary tangles and the amyloid-based neuritic plaques (Haroutunian et al., 1998, Matsson et al., 2000, Kawas et al., 2003).

Neurofibrillary tangles are composed of microtubule-associated hyperphosphorylated tau protein. Tau is associated with tubulin in the formation of microtubules. One function of microtubules is to provide points of attachment for chromosomes during cell division, which, if disrupted may result in an increased incidence of chromosome malsegregation and genomic instability (Iqbal et al., 1998, Petkova et al., 2002). The second histopathological feature of AD patients is the presence of amyloid-based neuritic plaques. $\beta$-amyloid peptide (A$\beta$42) originates from the aberrant proteolysis of the amyloid precursor protein (APP) (Petkova et al., 2002, Antzutkin et al., 2002). The APP gene APP is located on chromosome 21. Aneuploidy of chromosomes 17 and 21 are common hallmarks of AD and genomic instability (Thomas et al., Mutagenesis 2008).

AD is an age related disease associated with genomic instability. Telomere shortening was studied in lymphocytes and fibroblasts in AD and age related healthy controls (Panossian et al., 2003, Cawthorn et al., 2003). A study by Thomas using PCR revealed a trend of shorter telomeres in AD samples compared to age matched controls (Thomas et al., 2008). Shorter telomeres were detected in peripheral blood mononuclear cells from AD patients (Honig et al., 2006, Thomas et al., 2008, Lukens et al., 2009). Jenkins et al (Neuroscience Letters, 2008, 440(3):340-343) noted that absence of telomeres may indicate Alzheimer's disease/dementia status in older individuals with Down syndrome.

## Summary of the Disclosure

An aspect of the disclosure provides a method for evaluating cells derived from a subject suspected of having or having Alzheimer's disease or dementia comprising:

 a) the use of a test cell sample from the subject, the test cell sample comprising buccal cells,

 b) assaying the test cell sample to determine the telomeres organization signature of the test cell sample using three-dimensional (3D) analysis and quantitative fluorescence in situ hybridization (q-FISH), wherein determining the telomeres organization signature comprises detecting one or more of telomere numbers, telomere length and nuclear volume,

 c) comparing the test cell sample telomeres organization signature to one or more control telomeres organization reference signature, and

 d) identifying differences or similarities between the test cell sample telomeres organization signature and the one or more telomeres organization reference signature;

wherein an increase in the telomere numbers, a decrease in the telomere length and/or a decrease in the nuclear volume in the test cell sample telomeres organization signature compared to the reference telomeres organization signature is indicative the subject has Alzheimer's disease or dementia or an increased risk of developing Alzheimer's disease or dementia.

The Alzheimer's disease is optionally mild Alzheimer's disease, moderate Alzheimer's disease or severe Alzheimer's disease.

In an embodiment, a decrease of at least 10, 20, 30, 40 or 50% in the nuclear volume in the test sample telomeres organization signature compared to the reference telomeres organization signature is indicative the subject has Alzheimer's disease or dementia or an increased risk of developing Alzheimer's disease or dementia.

In an embodiment, determining the telomeres organization comprises determining telomere numbers, telomere length and cell nuclear volume.

Detecting one or more of an increase in the telomere numbers, a decrease in telomere length and a decrease in the nuclear volume in the test cell sample telomeres organization signature compared to the reference telomeres organization signature is for example indicative of Alzheimer's disease or dementia or an increased likelihood of developing Alzheimer's disease or dementia.

In another aspect of the disclosure, a method for evaluating cells derived from a subject suspected of having or having Alzheimer's disease or dementia is provided comprising:

 a) using a first test cell sample from the subject, the first test cell sample comprising buccal cells,

 b) using a second test cell sample from the subject, the second test cell sample obtained subsequently to the first test cell sample comprising buccal cells,

 c) assaying the first and second test cell samples to determine the telomeres organization signature of the first and second test cell samples using 3D analysis and q-FISH, wherein determining the telomeres organization signature comprises detecting one or more of telomere numbers, telomere length and nuclear volume,

 d) comparing the first test cell sample telomeres organization signature to the second test cell sample telomeres organization signature, and

 e) identifying differences or similarities between the first test cell sample telomeres organization signature and the second test cell sample telomeres organization signature;

wherein a difference in the telomeres organization signature of the second test cell sample compared to the telomeres organization signature of the first test cell sample is indicative the subject has progressing Alzheimer's disease or dementia or ameliorating Alzheimer's disease or dementia and a lack of difference in the telomeres organization signature of the second test cell sample compared to the telomeres organization signature of the first test cell sample is indicative of stable Alzheimer's disease or dementia, the difference being an increase in the telomere numbers, a decrease in the telomere length and/or a decrease in the nuclear volume.

Optionally, the difference in telomeres organization is telomere numbers and/or telomere length.

In another embodiment, determining the telomeres organization signature comprises detecting telomeres with a relative fluorescent intensity of (a) less than 20000 units, (b) 20001-40000 units and (c) greater than 40001 units.

In an embodiment, decreases in telomere length of short and mid-size and/or large telomeres is indicative of disease or an increased likelihood of disease (e.g. Alzheimer's, or dementia).

Optionally, the sample further comprises lymphocytes, leukocytes, peripheral blood mononuclear cells or fibroblast cells.

In another embodiment, the telomeres organization is determined on interphase telomeres.

Other features and advantages of the present disclosure will become apparent from the following detailed description. It should be understood, however, that the detailed description and the specific examples while indicating preferred embodiments of the disclosure are given by way of illustration only, since various changes and modifications within the spirit and scope of the disclosure will become apparent to those skilled in the art from this detailed description.

## Brief description of the drawings

An embodiment of the disclosure will now be described in relation to the drawings in which:

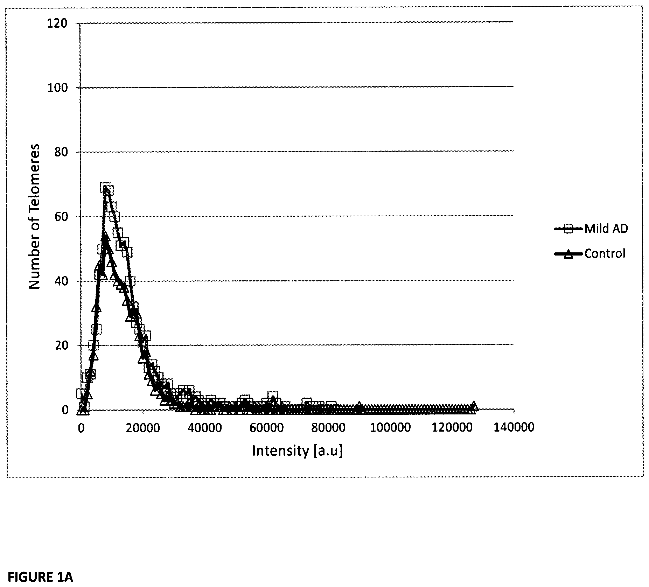

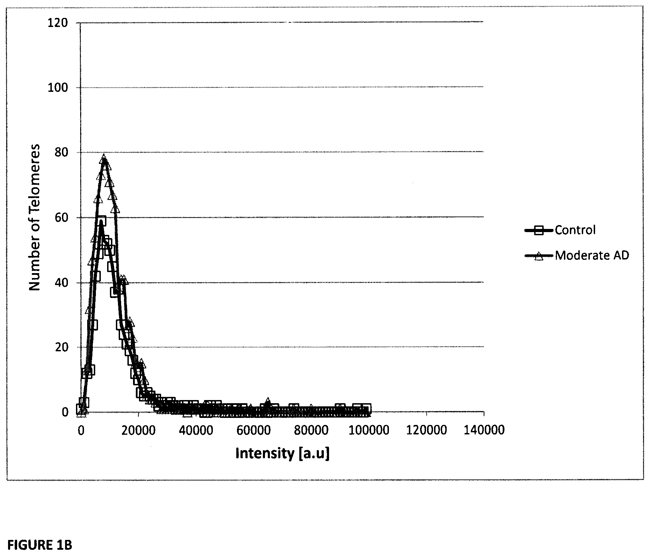

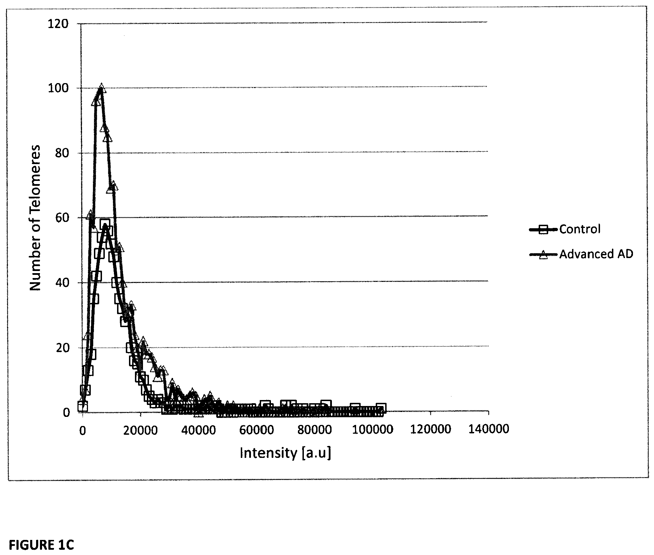

Figure 1. Telomere distribution according to their number and size in buccal cells of Alzheimer's patients (Figure 1A-Mild AD; Figure 1B-Moderate AD; Figure 1C -Severe AD) from different staging groups and their healthy controls. Results are based on 3D analysis of 30 cells from patients and representative controls. All three staging groups of Alzheimer's patients showed (grey line) significant increases (**A**-$p < .0001$; **B**-$p < .0001$; **C**-$p < .0001$) in number of telomeres as compared to healthy controls (black line). The mild, moderate and advanced AD patients' telomeres are also shorter compared to healthy controls ($A$-$p < .0001$; $B$-$p < .0001$; $C$-$p \ll 0.0247$).

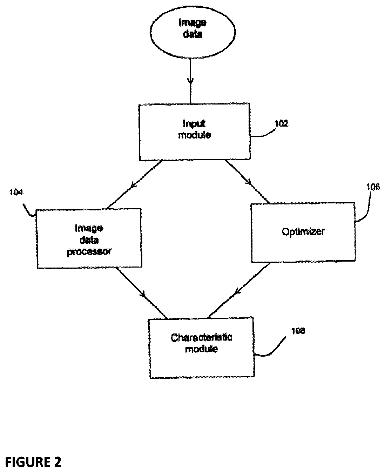

Figure 2. Block diagram of a system for characterizing a 3D organization of telomeres in interphase nuclei.

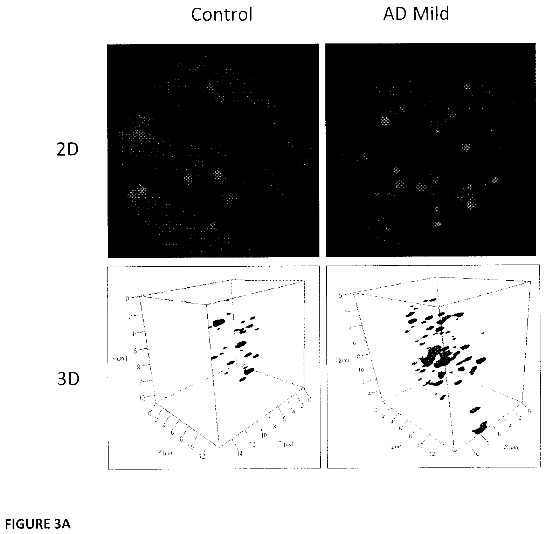

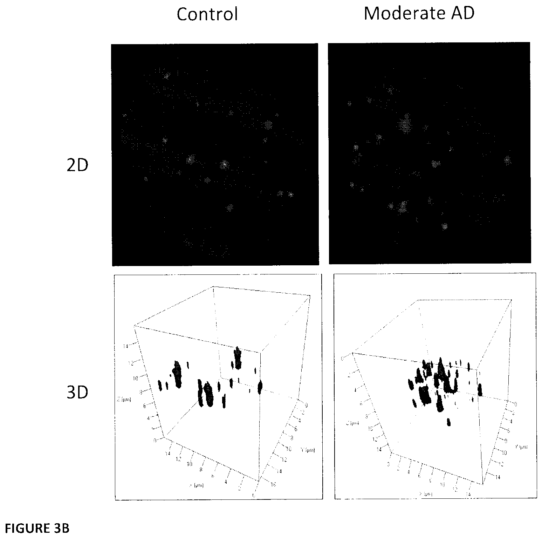

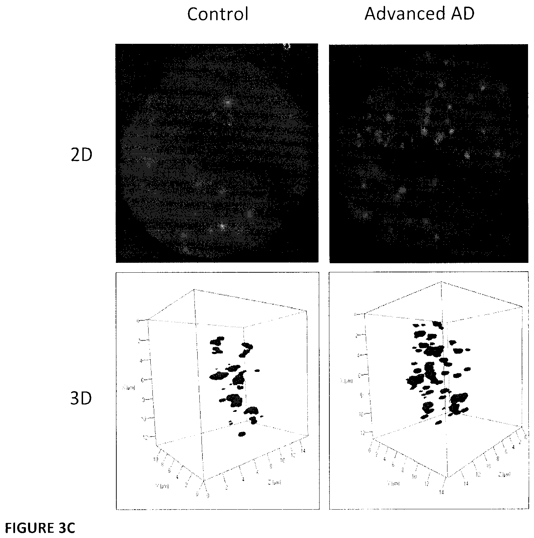

Figure 3. Figure 3 depicts 2D and 3D nuclear staining of telomeres and DNA in Buccal cells from Alzheimer's patients and a healthy control (Figure 3A-mild AD; Figure 3B-moderate AD; Figure3C-advanced AD)I. Representative 2D pictures showed significantly increased numbers of telomeres in each of the Alzheimer's staging groups compared to healthy controls. 3D images reveal not only an increased number of telomeres but also shorter telomeres in Alzheimer's patients.

## Detailed description of the Disclosure

The present disclosure will now be further described. In the following passages, different aspects are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

## I. Definitions

The term "Alzheimer's disease" as is known in the art used herein means a neurodegenerative condition resulting in neuronal death wherein patients show for example symptoms of impaired memory, judgment and decision-making among other cognitive disabilities, wherein patients are diagnosed on clinical grounds while excluding other causes of dementia, and includes, mild, moderate and severe (e.g. advanced) AD. AD patients are currently diagnosed on clinical grounds while excluding other causes of dementia. Diagnosis presently involves a comprehensive evaluation such as a complete health history, physical examination, neurological and mental status assessments, analysis of blood and urine, electrocardiogram, and possibly an imaging exam, such as CT or MRI. Conclusive AD diagnosis involves post mortem analysis. The two histopathological structures present within the brain that positively identify AD conclusively at post-mortem are the neurofibrillary tangles and the amyloid-based neuritic plaques (Haroutunian et al., 1998, Matsson et al., 2000, Kawas et al., 2003).

"Mild AD" as used herein in reference to .a patient means for example a patient with a Montreal Cognitive Assessment (MoCA) test (Nasreddine et al., 2005) score above 18/30; "Moderate AD" refers to for example a patient with a Mini-Mental State Exam (MMSE) (Folstein et al., 1975) score between 16/30 and 21/30 (inclusive); and "Severe AD" or "Advanced AD" which are used interchangeably, refers for example to a patient with an MMSE score < 16/30. Other comparable grading scales can also be used.

The term "buccal cells" or "BCs" as used herein means cells in the mouth cavity including for example buccal epithelial cells from the cheek.

The term "control" as used herein means any tissue, biological fluid or cell sample from one or more subjects not having Alzheimer's disease (AD) (e.g. control subjects) such as an age matched control or a value derived from such samples describing a telomeric organization parameter (e.g. determined from a sample from a control subject or group of control subjects). The value can be a threshold or cut off value, for example corresponding to number of telomeres, above which is associated with AD, or telomere length, below which is associated with AD. In embodiments where the severity of AD is being compared, the control can be a disease control for example mild AD. A subject with telomere parameters such as a decrease in length or increase in number compared to the disease control is identified as having moderate or severe AD. The control can be a value arising from population studies, theoretical models, or the characterization of control cells.

The term "age matched control" as used herein means a control that is within 15 years, 10 years, 5 years or 1 year of the test subject.

The phrase "characterizing telomeric organization of cells" as used herein means the application of a method comprising an algorithm to image data to determine at least one parameter of the telomeric organization, or optionally acquiring image data and the application of a method comprising an algorithm to image data to determine at least one parameter of the telomeric organization.

The phrase "determining telomeric organization of cells" as used herein means the application of a method to a sample which results in identifying at least one parameter that characterizes the telomeric organization. For example, parameters include telomere number in a cell, telomere length, and a/c ratio.

The term "sample" as used herein means any tissue, biological fluid or cell sample comprising chromosomal DNA containing cells (e.g. test cells) from a subject, including for example buccal cells, lymphocytes, leukocytes, peripheral blood mononuclear cells or fibroblast cells. The sample can also comprise brain tissue for example collected post mortem. The sample can be processed using methods known in the art. For example, buccal cells can be obtained by buccal swab using sterile swabs, smearing the buccal cells on microscope slides and storing the samples frozen and/or fixed, optionally using formaldehyde and stored until ready for processing.

As used herein, the term "cell" includes more than one cell or a plurality of cells or portions of cells. The term "test cell" is a cell from a subject that is suspected of having Alzheimer's disease and/or dementia. The term "control cell" is a suitable comparator cell e.g. a cell that is an age matched control. In one embodiment, a "test cell sample" comprises at least 5, 10, 15, 20, 25, 30, 40 or 50 cells.

The term "subject" as used herein refers to any member of the animal kingdom, preferably a human being.

The term "three dimensional (3D) analysis" as used herein means any technique that allows the 3D visualization and/or image analysis of cells, for example high resolution deconvolution microscopy, and can include one or more of 3D microscopy, image restoration or deconvolution, visualization and image analysis. An example of 3D image analysis is provided in Vermolen et al., 2005, and US. Patent No. 7,801,682, issued September 21, 2010 titled **Method of Monitoring Genomic**

**Instability Using 3D Microscopy and Analysis.**

The term "two dimensional (2D) analysis" as used herein means any technique that allows the 2D visualization and/or image analysis of cells, such as 2D microscopy and can include one or more of 2D microscopy, visualization and image analysis.

The terms "telomeric organization" and "telomeres organization" as used herein refers to the 3D arrangement of the telomeres during any phase of a cell cycle and includes such parameters as alignment (e.g. nuclear telomere distribution), state of aggregation, telomere numbers per cell and/or telomere sizes, a/c ratios and/or nuclear volumes. For example, fluorescent intensity is proportional to telomere size. Telomere size can be assessed by measuring fluorescent units (which are arbitrary units) as is demonstrated in the graphs of mild, moderate and severe AD compared to controls. "Telomeric organization" also refers to the size and shape of the telomeric disk, captured for example in an a/c ratio and which is the organized structure formed when the telomeres condense and align during the late G2 phase of the cell cycle. The term "state of aggregation" refers to the presence or absence of telomere aggregate(s) and/or the size and shape of the aggregates of telomeres. For example, telomeres with a relative fluorescent intensity (x-axis) ranging from 0-20,000 units are classified as short, with an intensity from 20,001-40,000 units as mid-sized, and with an intensity >40,001 units as large. Mid and large size telomeres can also be grouped together for example >20,001 units. As another example, telomere aggregates are defined as clusters of telomeres that are found in close association and cannot be further resolved as separate entities at an optical resolution limit of for example 200nm ($63\times$ oil) and 350 nm ($40\times$).

The term "telomeres organization signature" as used herein refers to a telomeric organization of a cell or average of a group of cells for example at least 5 cells, a least 10 cells, at least 15 cells, at least 20 cells, at least 25 cells or at least 30 cells and which can be used to classify the cell sample for example as normal or aberrant; Alzheimer's or non-Alzheimer's; progressing or stable; responsive to treatment or non-responsive to treatment. The criteria that define the differences include such parameters as alignment (e.g. nuclear telomere distribution), state of aggregation, telomere numbers per cell and/or telomere sizes, a/c ratios and/or nuclear volumes. The telomeres organization signature can be from a test cell sample or reference cell sample or samples.

The term "test cell sample telomeres organization signature" as used herein refers to a telomeres organization signature obtained from a cell or group of cells in a test sample, for example a cell from a subject that is suspected of having Alzheimer's disease or a risk of having Alzheimer's disease.

The term "reference telomeres organization signature" as used herein refers to a telomeres organization signature from a control or reference cell sample or derived therefrom. For example, a reference telomeres organization signature is optionally obtained from a cell sample from a subject or group of subjects that is known as not having Alzheimer's disease or a risk of having Alzheimer's disease (e.g. negative control) or that is known as having Alzheimer's disease (e.g. positive control).

The term "telomere length "as used herein refers to the relative fluorescent intensity of telomeres. For example, telomeres with a relative fluorescent intensity (x-axis) ranging from 0-20,000 units are classified as short, with an intensity from 20,001-40,000 units as mid-sized, and with an intensity >40,001 units as large (Knecht H, Sawan B, Lichtensztejn Z, Lichtensztejn D, Mai S.. Lab Invest. 2010;90(4):611-619).

The "difference in telomeric organization between the sample and the control and/or in the test cell compared to the control cell" or "differences or similarities between the test sample signature and the one or more control reference signatures" can be determined, for example by counting the number of telomeres in the cell, measuring the size or volume of any telomere or telomere aggregate, or measuring the alignment of the telomeres, and comparing the difference between the cells in the sample and the cells in the control. The differences in telomeric organization between the sample and the control can be measured and compared using individual cells or average values from a population of cells. The telomeres in a test cell may also be fragmented and therefore appear smaller than those in the control cell. Accordingly, a change or difference in telomeric organization in the test cell compared to the control cell can be determined by comparing parameters used to characterize the organization of telomeres. Such parameters are determined or obtained for example, using a system and/or method described herein below.

The term "short telomeres" as used herein means telomeres with a relative fluorescent intensity (x-axis) ranging from 0-20,000 units which are classified as short, the term "mid-sized telomeres" as used herein means telomeres with a relative fluorescent intensity (x-axis) ranging from 20,001-40,000 units which are classified as mid-sized and "large telomeres" as used herein means telomeres with a relative fluorescent intensity (x-axis) of >40,001 units which are classified as large.

The term "a/c ratio" as used herein describes the level to which the volume occupied by the telomeres is oblate. The larger it is, the more oblate (or disklike) is the shape of the volume occupied by the telomeres, while a/c = 1 means that this volume is spherical.

The term "nuclear volume" as used herein means the volume of a cell nucleus. Nuclear volume can be calculated according to the 3D nuclear 4', 6-diamidino-2-phenylindole staining (DAPI) protocol described in Vermolen BJ et al., (2005).

As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

"Palliating" a disease or disorder means that the extent and/or undesirable clinical manifestations of a disorder or a disease state are lessened and/or time course of the progression is slowed or lengthened, as compared to not treating the disorder.

In understanding the scope of the present disclosure, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Finally, terms of degree such as "substantially", "about" and "approximately" as used herein mean a reasonable amount of deviation of the modified term such that the end result is not significantly changed. These terms of degree should be construed as including a deviation of at least $\pm 5\%$ of the modified term if this deviation would not negate the meaning of the word it modifies.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." Further, it is to be understood that "a " "an," and "the" include plural referents unless the content clearly dictates otherwise. The term "about" means plus or minus 0.1 to 50%, 5-50%, or 10-40%, preferably 10-20%, more preferably 10% or 15%, of the number to which reference is being made.

## II. Methods

It is demonstrated herein using 3D analysis that the number of telomeres is increased and lengths of telomeres and nuclear volume are decreased in subjects with Alzheimer's disease compared to healthy controls. It is demonstrated for example that the changes in telomere numbers and lengths are sufficiently uniform to allow differentiation between AD patients and controls.

Differences were also seen with aphasia. Aphasia can result from head injury or stroke or develop from dementia.

3D nuclear imaging analysis is a very sensitive technique and as demonstrated herein can be used to measure not only telomere length but also telomere aggregates and numbers in the buccal cells, nuclear volume, distributions of telomeres in the nucleus from its center to periphery and a/c ratio. The methods described for example permit more parameters to be assessed providing more comprehensive diagnostic characteristic. In addition, as 3D analysis is very sensitive, diagnostic accuracy is improved.

As telomere numbers continue to increase and telomere length continues to reduce with progressive AD, it would seem predictable that a subject that has increased telomere numbers and decreased telomere lengths, but which does not meet the criteria for AD, for example using a mental exam, is at risk of developing AD (e.g. according to presently defined criteria).

An aspect of the disclosure provides a method for evaluating cells derived from a subject suspected of having or having Alzheimer's disease or dementia comprising:

a) the use of a test cell sample from the subject, the test cell sample comprising buccal cells,

b) assaying the test cell sample to determine the telomeres organization signature of the test cell sample using three-dimensional (3D) analysis and quantitative fluorescence in situ hybridization (q-FISH), wherein determining the telomeres organization signature comprises detecting one or more of telomere numbers, telomere length and nuclear volume,

c) comparing the test cell sample telomeres organization signature to one or more control telomeres organization reference signature, and

d) identifying differences or similarities between the test cell sample telomeres organization signature and the one or more telomeres organization reference signature;

wherein an increase in the telomere numbers, a decrease in the telomere length and/or a decrease in the nuclear volume in the test cell sample telomeres organization signature compared to the reference telomeres organization signature is indicative the subject has Alzheimer's disease or dementia or an increased risk of developing Alzheimer's disease or dementia.

The Alzheimer's disease is optionally mild Alzheimer's disease, moderate Alzheimer's disease or severe Alzheimer's disease.

An aspect provides a method for evaluating cells derived from a subject suspected of having or having Alzheimer's disease or dementia comprising:

a) using a first test cell sample from the subject, the first test cell sample comprising buccal cells,

b) using a second test cell sample from the subject, the second test cell sample obtained subsequently to the first test cell sample comprising buccal cells,

c) assaying the first and second test cell samples to determine the telomeres organization signature of the first and second test cell samples using 3D analysis and q-FISH, wherein determining the telomeres organization signature comprises detecting one or more of telomere numbers, telomere length and nuclear volume,

d) comparing the first test cell sample telomeres organization signature to the second test cell sample telomeres organization signature, and

e) identifying differences or similarities between the first test cell sample telomeres organization signature and the second test cell sample telomeres organization signature;

wherein a difference in the telomeres organization signature of the second test cells sample compared to the telomeres organization signature of the first test cells sample is indicative the subject has progressing Alzheimer's disease or dementia or ameliorating Alzheimer's disease or dementia and a lack of difference in the telomeres organization signature of the second test cell sample compared to the telomeres organization signature of the first test cell sample is indicative of stable Alzheimer's disease or dementia, the difference being an increase in the telomere numbers, a decrease in the telomere length and/or a decrease in the nuclear volume.

In an embodiment, determining the telomeric organization in the test sample cells and/or control comprises using quantitative fluorescence *in situ* hybridization (quantitative FISH or Q-FISH). For example, sample cells can be hybridized using a telomere PNA FISH probe. Digital images of the hybridized cells can for example be taken using a Zeiss AxioImager and images can be acquired for example by Axiovision (Zeiss) followed by constrained iterative deconvolution as described below and for example in Example 1. In an embodiment, determining and/or characterizing the telomeric organization in the test cell comprises using three dimensional (3D) analysis. Examples of 3D analysis are described below and in Vermolen et al 2005 and below.

A difference in telomeric organization is found for example when at least a parameter of the 3D organization is different compared to control cells. In an embodiment, the difference is an increased number of telomeres in the test sample cells compared to a control. In another embodiment, the difference is a decrease in the length of telomeres in the test sample cells compared to a control. For example, where the control is a healthy control, an increase in the number of telomeres and decrease in the length of telomeres is indicative the subject has Alzheimer's disease or an increased risk of developing Alzheimer's disease. The length of telomeres can for example be the average length of telomeres in a cell, or a number of cells. In an embodiment, the number of cells assessed is sufficient for statistical analysis. For example, at least 5 cells, 10 cells, 15 cells, 20 cells, 25 cells or 30 cells are analyzed for telomeric organization. The statistical tests that can be employed include for example Chi square test for telomere length, and Fisher's exact test for telomere numbers. ANOVA can also be used. In an embodiment, the statistical test used is a Student T test. In an embodiment, the increase (or decrease) is a statistically significant increase (or decrease). The increased risk for example can be expressed as an odd's ratio.

It is demonstrated for example that subjects with Alzheimer's disease have significant differences in short (e.g. low intensity), mid-sized (e.g. mid intensity) and large (high intensity) telomeres compared to normal and also according to severity of disease (e.g. mild, moderate and severe AD).

Low intensity is for example considered to be 0-20,000K relative fluorescent units, mid intensity is for example considered to be 20,001-40,000 relative fluorescent units and high intensity is considered to be >40,001 relative fluorescent units. Using these intensities, mild AD is significantly different from normal age-matched controls, and from moderate or severe AD.

Detecting one or more of an increase in the telomere numbers, a decrease in telomere length and a decrease in the nuclear volume in the test cell sample telomeres organization signature compared to the reference telomeres organization signature is for example indicative of Alzheimer's disease or an increased likelihood of developing Alzheimer's disease.

In an embodiment, the telomere number associated with AD is for example, greater than 60, greater than 70, greater than 80 or greater than 90.

In an embodiment, the telomere number associated with mild AD is about 60 to about 70, with moderate AD, about 70 to about 90 and advanced AD, greater than 90.

In an embodiment, the decrease in telomere intensity associated with AD is at least 10%, at least 20%, at least 30%, at least 50% decreased compared to a control. The decrease is optionally in telomeres having a fluorescence intensity within 0-20000 Units, 20001-40000 units and/or greater than 40001 units. Typically decreased in all three ranges are documented. In an embodiment, the decrease in telomere intensity associated with mild AD is at least 10%, or at least 20% decreased compared to control. In an embodiment, the decrease in telomere intensity associated with moderate AD is at least 10%, at least 20% or at least 30% decreased compared to control. In an embodiment, the decrease in telomere intensity associated with advanced AD is at least 10%, at least 20%, at least 30% or at least 40% decreased compared to control.

In an embodiment, the decrease in nuclear volume associated with AD is at least 10%, at least 20%, at least 30%, at least 50% decreased compared to a control. In an embodiment, the increase in the number of telomeres indicative of having Alzheimer's disease or an increased risk of developing Alzheimer's disease, is a increase of at least 5%, 10%, 15%, 20% or 25% compared to a control. In an embodiment, the increase in the number of telomeres is between about 10% and about 75% or between about 25% and 75% compared to control. In another embodiment, the decrease in the length of telomeres indicative of having Alzheimer's disease or an increased risk of developing Alzheimer's disease, is a decrease of at least 10%, 15%, 20%, 25%, 30%, 35%, 40%, 45% or 50% compared to a control. In an embodiment, the decrease in the length of telomeres is between about 10% and about 70% or between about 50% and 70% compared to control.

The control can for example be a threshold where in which subjects with a number of telomeres above the threshold and length of telomeres below the threshold are indicated as having Alzheimer's disease or an increased risk of Alzheimer's disease.

In an embodiment, the test sample comprises buccal cells, lymphocytes, leukocytes, peripheral blood mononuclear cells or fibroblast cells. In another embodiment, the test sample is brain tissue, for example collected post mortem.

In an embodiment, the Alzheimer's disease is mild Alzheimer's disease. In another embodiment, the Alzheimer's disease is moderate Alzheimer's disease. In yet another embodiment, the Alzheimer's disease is severe Alzheimer's disease.

As the telomeric alterations seen in samples increased with disease severity, the methods described herein can also be used to assess disease severity and/or for monitoring disease.

For example, subjects with severe Alzheimer's disease had an increased number of telomeres and shorter telomeres compared to healthy age matched controls (see for example Figure 1C).

The method can also be used to monitor treatment therapy.

For example, if a sample obtained after treatment indicates that the cell telomere lengths decreased and/or numbers are increased compared to the sample obtained prior to the one or more treatments, the subject is predicted to not be responding to treatment. If the telomere lengths and/or numbers are stabilized and/or telomere lengths are increased and/or numbers are decreased, the subject is predicted to be responding to the treatment.

In an embodiment the telomere organization is determined for interphase telomeres.

In one embodiment, determining the telomeres organization signature comprises detecting telomere numbers. In another embodiment, determining the telomeres organization signature comprises detecting telomere length. In yet another embodiment, determining the telomeres organization signature comprises detecting telomere numbers and telomere length.

Also disclosed is use of the methods described for selecting a treatment, wherein a subject is monitored for response to a treatment and treatment is continued if responding or a new treatment is selected if not responding.

The methods can also be used for example to differentiate subjects in clinical trials testing new therapies.

In an embodiment, an automated method is used for example Teloscan™ (Klewes et al 2011).

**a) Method of characterizing 3D organization of patient samples**

Methods and systems for determining the 3D organization of telomeres are described in US Patent No. 7,801,682, issued September 21, 2010 titled **Method of Monitoring Genomic Instability Using 3D Microscopy and Analysis.**

In an embodiment the method for characterizing a 3D organization of telomeres comprises:

(i) inputting image data of the 3D organization of telomeres;
(ii) processing the image data using an image data processor to find a set of coordinates $\{(x_i,y_i,z_i)\}$, $i = 1,...,N$, where $(x_i,y_i,z_i)$ is a position of the ith telomere;
(iii) finding a plane that is closest to the set of coordinates; and
(iv) finding a set of distances $\{d_i\}$, $i = 1,...,N$, where $d_i$ is the distance between $(x_i,y_i,z_i)$ and the plane, wherein the set $\{d_i\}$ is utilized to characterize the 3D organization.

Figure 2 shows a block diagram of a system 100 for characterizing a 3D organization of telomeres. The system 100 includes an input module 102, an image data processor 104, an optimizer 106 and a characteristic module 108.

An input module 102 can be used to input image data of the 3D organization of telomeres. The input module 102 includes appropriate hardware and/or software, such as a CD-ROM and CD-ROM reader, DVD and DVDreader or other data storage and reading means including for example external hard drives. The inputting performed by the input module 102 need not be from outside the system 100 to inside the system 100. Rather, in some embodiments, the inputting of data may describe the transfer of data from a permanent storage medium within the system 100, such as a hard disk of the system 100, to a volatile storage medium of the system 100, such as RAM.

The image data can be obtained using regular or confocal microscopy and can include the intensities of one or more colors at pixels (totaling, for example, 300x300 or 500x500) that comprise an image of a nucleus. The image data can also be grey level image data of a nucleus that has been appropriately stained to highlight telomeres. Several images (on the order of 100) are obtained corresponding to slices along a particular axis. Thus, the image data may correspond to a total of about $2.5 \times 10^7$ pixels. In one embodiment, the slices may be on the order of 100 nanometers apart. In this manner, the image data accounts for the 3D quality of the organization of telomeres. In addition, the confocal microscope is able to obtain the intensity of two colors, for example blue and green, of the nucleus at every pixel imaged, thereby doubling the amount of data points.

To obtain an image of telomeres, a stain such as DAPI (4',6-diamidino-2-phenylindole) can be used to preferentially mark the heterochromatin material that comprises DNA. A second stain, such as cy3, together with an appropriate label, such as PNA telomere probe, can be used to mark the telomeric portion of the heterochromatin material.

To improve the quality of the image data, various techniques can be brought to bear as known to those of ordinary skill, such as constrained iterative deconvolution of the image data to improve resolution. Such constrained iterative deconvolution may not be required if confocal, instead of regular, microscopy is used as the image data may be of superior resolution. In addition, other instruments, such as an apotome, may be used to improve the quality of the image.

In an embodiment, the 3D organization is characterized by specifying at least one of $\bar{d}$ and $\sigma$, where $\bar{d}$ is the average distance of the set of distances, and $\sigma$ is the standard deviation of the set of distances.

In another embodiment, the characterization is used to monitor and/or diagnose Alzheimer's disease by comparing the at least one of $\bar{d}$ and $\sigma$ to a corresponding control value.

In an embodiment, the method of characterizing a 3D organization of telomeres comprises:

(i) inputting image data of the 3D organization of telomeres; and
(ii) using an image data processor for finding a three dimensional geometrical shape that best encompasses the 3D organization, wherein the geometrical shape is an ellipsoid having principal axes $a_1, a_2,$ and $a_3$ and wherein said shape is used to characterize the 3D organization.

The image data processor 104 processes the image data to find a set of coordinates $\{(x_i, y_i, z_i)\}$, $i = 1,...,N$, where $(x_i, y_i, z_i)$ is a position of the ith telomere. For this purpose, the image data processor 104 identifies "blobs" within the image data that can be identified as a telomere using a segmentation process. Each blob identified as a telomere has a non-negligible volume (for example, a small telomere may have a volume of 4x4x4 pixels, a large one a volume of 10x10x10, where the size of the nucleus may be approximately 200x200x100 pixels). There is some freedom, therefore, in choosing "the position" of the telomere. One possibility is to choose for this position the center of gravity of the telomere, or more generally, the telomere organization.

In an embodiment, the ellipsoid is an oblate spheroid with $a_1$ approximately equal to $a_2$.

In an embodiment, an oblateness ratio, $a_3 / a_1$ or $a_1 / a_3$, is used to characterize the 3D organization.

In an embodiment, the method for characterizing a 3D organization of telomeres comprises:

(i) inputting image data of the 3D organization of telomeres and
(ii) obtaining from the image data using an image data processor at least one of a set of intensities $\{I_i\}$, a set of volumes $\{V_i\}$ and a set of three dimensions $\{(Dx_i, Dy_i, Dz_i)\}$, $i = 1,...,N$, where $I_i$ is a total or average intensity, $V_i$ is a volume, and $(Dx_i, Dy_i, Dz_i)$ are principle axes of an ellipsoid describing the ith telomere, respectively, wherein the at least one is utilized to characterize the 3D organization.

In an embodiment, said characterization is used to monitor and/or diagnose Alzheimer's disease and/or treatment efficacy by comparing a quantity obtained from at least one to a control value.

In an embodiment, the quantity is an average of the members of $\{I_i\}$, $\{V_i\}$ or $(Dx_i, Dy_i, Dz_i)$.

In an embodiment, the method for characterizing a 3D organization of telomeres comprises:

(i) obtaining image data of the 3D organization of telomeres obtained using a microscope;
(ii) inputting the image data of the 3D organization of telomeres obtained using the microscope; and
(iii) finding a parameter of the 3D organization that measures a deviation of the 3D organization from a planar arrangement, the deviation used to characterize the 3D organization.

In yet another embodiment, the method for characterizing a 3D organization of telomeres of sample cells comprises:

(i) obtaining image data of the 3D organization of telomeres obtained using a microscope;
(ii) inputting the image data of the 3D organization of telomeres;
(iii) processing the image data to find a set of coordinates $\{(x_i, y_i, z_i)\}$, $i = 1,...,N$, where $(x_i, y_i, z_i)$ is a position of the ith telomere;
(iv) finding a plane that is closest to the set of coordinates;
(v) finding a set of distances $\{d_i\}$, $i = 1,...,N$, where $d_i$ is the distance between $(x_i, y_i, z_i)$ and the plane, wherein the set $\{d_i\}$ is utilized to characterize the 3D organization; and
(vi) visually displaying the 3D organization of the telomeres.

In an embodiment, the method for characterizing a 3D organization of telomeres of sample cells is performed on a system for characterizing a 3D organization of telomeres.

In an embodiment, the system comprises:

(i) an input module for inputting image data of the 3D organization of telomeres;
(ii) an image data processor for processing the image data to find a set of coordinates $\{(x_i, y_i, z_i)\}$, $i = 1,...,N$, where $(x_i, y_i, z_i)$ is a position of the ith telomere;
(iii) an optimizer for finding a plane that is closest to the set of coordinates; and
(iv) a characteristic module for finding a set of distances $\{d_i\}$, $i = 1,...,N$, where $d_i$ is the distance between $(x_i, y_i, z_i)$ and the plane, wherein the set $\{d_i\}$ is utilized to characterize the 3D organization.

The optimizer 106 finds a plane $P^{min}$ that is closest to the set of coordinates. To find the closest plane, the distance $D_i$ between the location of the ith telomere, $(x_i, y_i, z_i)$, and the plane given by $ax + by + cz = 0$ is considered:

$$D_i = \frac{ax_i + by_i + cz_i}{\sqrt{a^2 + b^2 + c^2}}.$$

The optimizer 106 finds the parameters a,b,c,d that minimize the function $\sum_{i=1}^{N} D_i(a,b,c,d)$.

The characteristic module 108 proceeds to find at least one parameter that can be used to characterize the 3D organization of telomere". "Parameters used to characterize the organization of telomeres" include:

1) A set of distances $\{d_i\}$, $i = 1,...,N$, where $d_i$ is the distance between $(x_i, y_i, z_i)$ and the plane $P^{min}$.

2) $\overline{d}$ and $\sigma$, the average distance and standard deviation of the set of distances $\{d_i\}$:

$$\overline{d} = \frac{1}{N} \sum_{i=1}^{N} d_i,$$

and

$$\sigma^2 = \sum_{i=1}^{N} \frac{(d_i - \overline{d})^2}{N},$$

respectively.

3) A three dimensional geometrical shape that best encompasses the 3D organization. For example, the geometrical shape can be the ellipsoid, having principal axes $a_1, a_2$, and $a_3$, that best encompasses the 3D organization of the telomeres. Several definitions of "best encompasses" can be used. For example, the ellipsoid that best encompasses the telomeres can be defined as the ellipsoid of smallest volume that encloses a certain fraction (e.g., 100%) of the telomeres. If a set of more than one ellipsoid fulfills this condition, other restrictions can be used to reduce the set to just one ellipsoid, such as further requiring the ellipsoid to have the smallest largest ratio of principle axes (i.e., the "most circle-like" ellipsoid). It should be understood that other definitions of "best encompasses" the telomeres can be used. It has been observed that the ellipsoid that best encompasses the telomeres often approximates an oblate spheroid with $a_1$ approximately equal to $a_2$. In such case, it is sufficient to specify just $a_2$ and $a_3$. Alternatively, an oblateness ratio, $a_3 / a_1$ or $a_1 / a_3$, can be used to characterize the oblate spheroid describing the organization of the telomeres.

4) A set of volumes $\{V_i\}$, where $V_i$ is the volume of the ith telomere.

5) A set of three dimensions $\{(Dx_i, Dy_i, Dz_i)\}$, $i = 1,...,N$, where $(Dx_i, Dy_i, Dz_i)$ are principle axes of an ellipsoid describing the ith telomere.

6) A set of intensities $\{I_i\}$, $i = 1,...,N$, where $I_i$ is the total intensity of the ith telomere. (In other embodiments, instead of the total intensity, the average intensity of each telomere can be computed.) That is, if the ith telomere is associated with K pixels, then

$$I_i = \sum_{j=1}^{K} I_{i,j}$$

where $I_{i,j}$ is the intensity of the jth pixel of the ith telomere.

In the last three cases, the sets can be used to calculate statistical measures such as an average, a median or a standard deviation.

The parameters 1-5 outlined above characterize the 3D organization of the telomeres by focusing on the geometrical structure of the telomeres. Parameters 1 and 2 are motivated by the finding that, especially during the late G2 phase of the cell cycle, telomeres tend to lie on a plane. Parameters 1 and 2 measure deviations of telomeres from a planar arrangement.

Parameter 3 attempts to describe, with features, such as the three principal axes of an ellipsoid or the oblateness ratio, the overall shape of the 3D organization. While parameters 1-3 are global geometric characteristics, dealing with the overall shape of the organization, parameters 4 and 5 are local geometric characteristics in the sense that they involve the geometry of each individual telomere.

The final parameter is also local, involving the intensity of each individual telomere.

In an embodiment, the 3D organization is characterized by specifying at least one of $\bar{d}$ and $\sigma$, where $\bar{d}$ is the average distance of the set of distances, and $\sigma$ is the standard deviation of the set of distances.

In an embodiment, the system further comprises a diagnosis module for comparing the at least one of $\bar{d}$ and $\sigma$ to a corresponding standard value to monitor or diagnose Alzheimer's disease.

In another embodiment, the method for characterizing a 3D organization of telomeres in the sample comprises:

(i) inputting image data of the 3D organization of telomeres; and
(ii) using an image data processor for finding a parameter of the 3D organization that measures a deviation of the 3D organization from a planar arrangement, the deviation used to characterize the 3D organization.

In an embodiment, a system is used for characterizing a 3D organization of telomeres in the sample, the system comprising

(i) an input module for inputting image data of the 3D organization of telomeres;
(ii) an image data processor for processing the image data to find a set of coordinates $\{(x_i,y_i,z_i)\}$, $i = 1,...,N$, where $(x_i,y_i,z_i)$ is a position of the ith telomere; and
(iii) a characteristic module for finding a parameter of the distribution that measures a deviation of the distribution from a planar arrangement, the deviation used to characterize the 3D organization.

In an embodiment, the method for characterizing a 3D organization of telomeres comprises:

(i) obtaining image data of the 3D organization of telomeres obtained using a microscope;
(ii) inputting the image data of the 3D organization of telomeres obtained using the microscope;
(iii) processing the image data to find a set of coordinates $\{(x_i,y_i,z_i)\}$, $i = 1,...,N$, where $(x_i,y_i,z_i)$ is a position of the ith telomere;
(iv) finding a plane that is closest to the set of coordinates; and
(v) finding a set of distances $\{d_i\}$, $i = 1,...,N$, where $d_i$ is the distance between $(x_i,y_i,z_i)$ and the plane, wherein the set $\{d_i\}$ is utilized to characterize the 3D organization.

In another embodiment, the method of characterizing a 3D organization of telomeres, comprises:

(i) obtaining image data of the 3D organization of telomeres obtained using a microscope;
(ii) inputting the image data of the 3D organization of telomeres obtained using the microscope; and
(iii) finding a three dimensional geometrical shape that best encompasses the 3D organization, wherein the geometrical shape is an ellipsoid having principal axes $a_1,a_2$, and $a_3$ and wherein said shape is used to characterize the 3D organization.

In another embodiment, the method for characterizing a 3D organization of telomeres, comprises:

(i) obtaining image data of the 3D organization of telomeres obtained using a microscope;
(ii) inputting the image data of the 3D organization of telomeres obtained using the microscope; and
(iii) obtaining from the image data at least one of a set of intensities $\{I_i\}$, a set of volumes $\{V_i\}$ and a set of three dimensions $\{(Dx_i, Dy_i, Dz_i)\}$, $i = 1,...,N$, where $I_i$ is a total or average intensity, $V_i$ is a volume, and $(Dx_i, Dy_i, Dz_i)$ are principle axes of an ellipsoid describing the ith telomere, respectively, wherein the at least one is utilized to characterize the 3D organization.

In an embodiment, determining the 3D organization of telomeres and comparing to a control is a computer implemented method.

In an embodiment, the computer implemented method is TeloVew In another embodiment, the computer implemented method is TeloScan.

The following non-limiting examples are illustrative of the present disclosure:

## Examples

## Example 1

One of the aims of this study was to investigate changes in the three-dimensional (3D) nuclear architecture in AD patients and age related healthy controls using 3D quantitative fluorescence *in situ* hybridization (3D Q-FISH) to determine if there were any differences in telomere number, length, aggregates and cell cycle profiles represented by a/c ratios (Vermolen et al., 2005) in AD patients compared to healthy age-matched controls.

Fifty-nine patients with AD (ranging in stage from mild to severe) and their fifty-nine cognitively normal age-matched caregivers were included in this study. Buccal cells (BCs) were used in the study because they have a number of advantages; not only can samples be collected non-invasively, but BCs also originate from the neuro-ectoderm, which is where brain tissue is derived from.

BCs were used previously to study telomere length in AD patients. A study using PCR showed that BCs have significantly shorter telomere lengths than age matched healthy controls (Thomas et al., 2008). However, the authors of that study looked only at telomere length using PCR technique and did not investigate the 3D nuclear telomere organization in AD. Further, Thomas et al. (2008) teaches that, for example, white blood cell DNA is potentially a more sensitive measure of differences in telomere length between AD cases and controls compared to buccal cells.

The study described herein indicates that the 3D Q-FISH technique is sensitive for analysis of telomere characteristics in BC samples of AD patients. To process and analyze the images of 3D nuclei of the samples, a software program called TeloView (Vermolen et al., 2005) was used. TeloView is designed to process the results of 3D FISH images from interphase cell nuclei. It segments telomeric signals and localizes them.

In the presently described study, the association between telomere length in cells obtained from buccal swabs from AD and healthy controls was examined. A significant difference in nuclear organizations of AD samples to controls is shown.

As demonstrated herein, it was found that 3D nuclear imaging is a very sensitive technique in which not only telomere length can be measured, but also telomere aggregates and numbers in the BCs, nuclear volume, distributions of telomeres in the nucleus from its center to periphery and a/c ratio. More parameters using this system can also be looked at.

## Material and Methods:

## Subjects

In the study, fifty-nine patients with Alzheimer's Disease (AD) attending the Queen's University Memory Clinics were compared to their fifty-nine age matched (+/- 5 years) cognitively normal caregivers according to the following parameters: telomere aggregation, telomere length, a/c ratio, nuclear volume and Fisher's exact test for telomere numbers. Diagnosis of AD was made according to the National Institute of Neurological and Communicative Disorders and Stroke, and the Alzheimer's Disease and Related Disorders Association (NINCDS-ADRDA) Alzheimer's criteria (McKhann et al., 1984) and all patients had been on standard treatment according to the Canadian Guidelines for the Treatment of Dementia. Patients were followed at the Memory Clinics and the dementia staged as mild, moderate or severe, according to their scores in the Montreal Cognitive Assessment test (MoCA) (Nasreddine et al., 2005) and/or the Mini-Mental State Exam (MMSE) (Folstein et al., 1975). Patients with mild AD (n=31) had MoCA scores above 18/30. Patients with moderate AD (n=12) had MMSE scores between 16/30 and 21/30 (inclusive). Patients with advanced AD disease (n=16) (e.g. severe disease) had MMSE scores < 16/30.

**Subject characteristics**

**Table 1**

| Number of patients enrolled in study | Stage of AD |
|---|---|
| 31 | Mild AD |
| 12 | Moderate AD |
| 16 | Advanced AD (e.g. severe AD) |
| 1 | Aphasia |
| Total 59 AD | |

**IN TOTAL**

31 Mild + 31 Controls
12 Moderate + 12 Controls
16 Advanced + 16 Controls

(59 Patients + 59 Controls)

**Buccal swabs**

Buccal swabs were obtained in duplicate, at the regular clinic visits, using sterile Epicentre Biotechnologies swabs. Samples from each swab were smeared on VWR micro slides and kept frozen at -20° C for 1 to 3 months until Q-FISH analysis.

**3D-in situ hybridization**

Cells were fixed with 3.7% formaldehyde (Fisher). Hybridization was done using a DAKO Cy3 telomere PNA probe (DAKO, Glostrup, Denmark). Three-dimensional telomere quantitative FISH (3D-Q-FISH) was performed as described previously (Knecht et al., 2009).

**Quantitative image Analysis**

Digital images were taken using a Zeiss AxioImager Z1 with a cooled AxioCam HR B&W, DAPI, Cy3 filters in combination with a Planapo 63x/1.4 oil objective lens. Images were acquired using AXIOVISION 4.8 (Zeiss) in multichannel mode followed by constrained iterative deconvolution (Schaefer et al., 2001). For every fluorochrome, 80 images stacks were acquired with a sampling distance of 200 nm along the z and 102 nm in the xy direction. Thirty interphase nuclei from each AD sample, as well as healthy control were analyzed. All results were analyzed statistically. Quantitation of 3D nuclear telomeric signals was performed using TeloView (Vermolen et al., 2005). After finding the threshold for the telomeres, the program analyzed the center of intensities for every object resulting in a set of coordinates ($x, y, z$). The integrated intensity of each telomere that is proportional in length was calculated.

**Statistics**

3D-Q-FISH results were analyzed with Fisher's exact test for telomere numbers and Chi square test for telomere length to determine significant differences between the AD groups and healthy age matched controls. A P value <0.05 was considered statistically significant.

## Results

Using 3D Quantitative Fluorescent in situ Hybridization (3D Q-FISH), it is possible to determine if there are differences in telomere number, length, telomeric aggregates, nuclear volume and cell cycle profiles represented by a/c ratios in AD patients compared to healthy age-matched controls. The telomeric profiles can also be used further categorize disease stages of patients as either Mild, Moderate, or Advanced Alzheimer's. Fifty-nine patients with ranging disease stages of AD and their fifty-nine cognitively normal age-matched caregivers were included in this double-blind pilot study.

**Increased number of telomeres and decreased telomere length in BCs from AD patients.**

Using the Teloview® software, the total number of telomeres of each patient as well as their healthy control was analyzed. The results of each sample were then graphed against their respective telomere lengths (Figures 1A-1C).

Figure 1A is a representative graph of patients identified with mild AD compared to age-matched healthy controls. The data revealed a statistically significant increased number of telomere signals in samples from Mild AD patients compared to controls ($p < 0.0001$), and a decrease in length of telomeres in Mild AD samples as compared to controls ($p < 0.0001$). The number of telomeres for the Mild AD patients was consistently found to range between 60 and 70 telomeres, whereas in the controls, the range varied between 40-60 telomeres.

Figure 1B is a representative graph of patients identified with Moderate AD compared to age-matched healthy controls. Moderate AD also shows an increasing number of telomeres, ranging between 70-90 ($p < 0.0001$) while showing a decrease in telomeric length ($p < 0.0001$) compared to healthy age matched controls.

Figure 1C illustrates the results for AD patients identified having Advanced Alzheimers. The data revealed a statistically significant higher number of telomere signals in samples from Advanced AD patients compared to controls ($p < 0.0001$), and a decrease in length of telomeres in Advanced AD samples as compared to controls ($p < 0.0247$). The number of telomeres for the Advanced AD patients was consistently found to be over 90.

Overall, the results show that AD patients have an increasing number of telomeres as their disease progresses to a more severe stage, with the advanced stage showing the highest numbers of telomeres. The age-matched controls however, tend to remain within the normal range (40-60 telomeres, de Vos et al., 2009) regardless of the control's age or gender.

The following table summarizes the results:

**Table 2: Ranges of telomeric values for healthy controls, as well as Mild, Moderate and Advanced Alzheimer patients using 3D Q-FISH analysis and 3D Imaging Software on cells obtained from Buccal Swabs.**

| Disease Stage | Number of Telomeres |
|---|---|
| None (Control) | 40-60 |
| Mild | 60-70 |
| Moderate | 70-90 |
| Advanced | > 90 |

## Discussion

Alzheimer's disease (AD) is a progressive degenerative disorder of the brain (Kawas et al., 2003, Matson et al. 2004). Advanced age is the major factor contributing to increased risk of developing AD (Aubert et al., 2007, Thomas et al., 2007). Telomeres have an important function in cell fate and aging. Telomere repeats progressively shorten after every cell division therefore telomere length may be used as a marker of a cell's replicative history (Allsopp et al., 1992). Alzheimer's diagnosis needs better markers for a quick and non-invasive assessment of the patients. There is no single test that can definitively diagnose Alzheimer's disease "in vivo". Therefore finding new biological markers is important for a better understanding of AD, its diagnosis and treatment. The investigations described herein show a strong relationship between AD and telomere lengths in BCs. An altered nuclear architecture of telomeres in AD has been shown herein, which is not seen in healthy controls.

The aim of this study was to investigate the 3D nuclear organization of telomeres obtained from AD patients from BCs. Using TeloView, telomere numbers, their lengths, percentage of aggregates and a/c ratio were analyzed (Vermolen et al., 2005). Results were compared to those obtained in healthy age matched controls. Three groups of AD patients were enrolled; mild, moderate and severe. Significantly lower telomere length in BCs were found in all three groups of AD patients as compared to age matched healthy controls (Table 2, Fig.1). The presently described studies using 3D nuclear imaging also revealed higher numbers of telomeres in AD patients (Fig. 1). It is believed that this is the first time that an association between number of telomeres and AD has been shown.

It has been demonstrated herein that there were no significant changes in numbers of telomere aggregates (TA) in AD groups as compared to aged matched healthy controls. TAs are seen in tumor cells (Mai and Garini 2005, 2006). TAs are aberrations in the nuclear organization. When TAs form and chromosome fusions occur, breakage/fusion/bridge (BBF) cycles result and the genetic information of the chromosomes will be remodeled (Louis et al. 2005). TA formation is independent of telomere size or telomerase activity (Chuang et al., 2003). While not wishing to be limited by theory, a lack of differences in TA formations in ADs and healthy aged matched controls may suggest that an altered mechanism of cell divisions is not the main cause of genetic changes in ADs as it is in cancer cells.

It has also been reported that 3D nuclear imaging is a very sensitive technique in which not only telomere length can be measured, but also telomere aggregates and numbers (Vermolen et al., 2005). The automation of 3D scanning for telomere signatures in interphase nuclei based on 3D-FISH has been described in relation to tumor cell detection (Klewes et al., 2011). The automated scanning, TeloScan, is suitable for large series of samples and sample sizes. The sensitivity of this automation for tumor cell detection has been defined; it has been shown to detect one aberrant tumor cell in 1,000 normal cells. The automation allows for a throughput of about 10,000 to 15,000 cells within one hour using the 40× objective. It will be tested if the same tool could be used for telomere analysis in AD patients. This would allow for large scale and faster detection of the AD samples. The data from the presently described study suggest that the numbers of telomeres are much higher and the length of telomeres are shorter in severe AD compared to moderate AD and mild AD.

Moreover, results described herein show that telomere intensity and number in patients with AD in all stages was significantly different from the normal controls. Telomeric aggregate and a/c ratios were also looked at. No changes in aggregate formation and numbers in the BC samples were noticed.

### Example 2

**Purpose**

Telomeres are linear repeats of two thymidine, an adenine and three glycine residues capping human chromosomes. They maintain chromosomal integrity and prevent chromosomal instability. Telomeres shorten progressively with each cell division and, therefore, with age. The main aim of the study described herein was to analyze the three-dimensional (3D) architecture of telomeres in AD patients compared to age-matched normal controls, and the feasibility of obtaining cells from buccal swabs for 3D analysis. 3D analysis allows for quantification of telomere numbers, length and aggregates. Buccal swabs were chosen because cells derive from the neuroectoderm from which brain tissue also originates, and they can be collected non-invasively. Previous studies have only investigated telomere length in different types of cell with conflicting results.

**Methods**

Fifty-nine patients with AD (stage mild to severe) and fifty-nine cognitively normal age-matched controls were included in this present study. Patients' caregivers served as normal controls. Patients were diagnosed and disease staged following standard procedures. Cells were obtained from buccal swabs using sterile Epicentre Biotechnologies swabs at follow-up memory clinic visits, smeared on VWR micro slides and frozen at -20° C until processing for telomere analysis. Quantitative fluorescence in situ hybridization (Q-FISH) technique was used for telomere numbers and length analysis in 30 interphase cells/person. Digital images were taken using Zeiss AxioImager Z1 with a cooled AxioCam HR B&W, DAPI, Cy3 filters in combination with a Planapo 63x/1.4 oil objective lens. Images were acquired using AXIOVISION 4.8 (Zeiss) in multichannel mode followed by constrained iterative deconvolution. For every fluorochrome, 80 images stacks were acquired with a sampling distance of 200 nm along the z, and 102 nm in the xy axis. Quantitation of 3D nuclear telomeric signals was performed using TeloView. Differences in telomere intensity between AD patients stratified by disease stage and their normal controls, were analyzed by Fisher exact test (number of telomeres) and Chi-Square (telomere length). Visual inspection of the images permitted analysis of telomere aggregates. A $p<.05$ was considered significant.

**Results**

It was found that patients with AD (all stages from mild to severe) had significantly more number of telomeres and significantly shorter telomeres than the control subjects (range from $p<.001$ to $p<.0001$). There was no difference in telomere aggregates between AD patients and controls. Telomere aggregates are found in some types of cancer.

**Conclusions**

Cells obtained from smeared buccal swabs are suitable for 3D analysis of telomeres in patients with AD and normal controls, allowing for further characterization of telomeres in this disease. In the study described herein, subjects with AD, at any stage of the disease had more and shorter telomeres in their buccal cells when compared to their age-matched controls, but no difference in aggregates.

**Example 3**

The following tables contain data relating to patients with aphasia (Table 3), mild AD (Table 4), moderate AD (Table 5) and severe AD (Table 6) as compared to controls. Further details regarding the AD patients are provided in Example 4. Statistical analysis for each sample set is found below the dataset. The analysis demonstrates that AD patients (mild, moderate and severe) have statistically different short, mid-sized and long telomeres compared to age matched controls.

For each of Tables 2-6, the three numbers corresponding to the control or patient for each intensity range represent the following:

The first row of numbers represents the frequency (i.e how many signals were in those particular ranges). The second row represents the row percentage (e.g the second row adds up to 100%). This row shows what percentage of the total signals were in the particular ranges. E.g., for Table 4 (mild AD), 1.47 % of the signals were under 20000, 4.02% in the mid range, and 94.52% above 40001. The third row of numbers is the column percentages. The third row of numbers from the control and patient adds up to 100%.

**Table 3: Aphasia (sample size = 3519)**

| Table of case/cntl by Intensity | | | | |
|---|---|---|---|---|
| **casecntl** | **Intensity(Intensity) x 10** | | | |
| **Frequency Row Pct Col Pct** | **<2000** | **2001-4000** | **>4001** | **Total** |
| **control** | 354 | 700 | 459 | 1513 |
| | 23.40 | 46.27 | 30.34 | |
| | 47.20 | 44.05 | 38.90 | |
| **patient** | 396 | 889 | 721 | 2006 |
| | 19.74 | 44.32 | 35.94 | |
| | 52.80 | 55.95 | 61.10 | |
| **Total** | 750 | 1589 | 1180 | 3519 |

*Statistics for Table of case/cntl by Intensity*

**Table 4: Mild AD - sample size: 2818**

| Statistic | DF | Value | Prob |
|---|---|---|---|
| **Chi-Square** | 2 | 14.2165 | 0.0008 |
| **Likelihood Ratio Chi-Square** | 2 | 14.2519 | 0.0008 |
| **Mantel-Haenszel Chi-Square** | 1 | 14.2120 | 0.0002 |
| **Phi Coefficient** | | 0.0636 | |
| **Contingency Coefficient** | | 0.0634 | |
| **Cramer's V** | | 0.0636 | |

| Table of casecntl by Intensity | | | | |
|---|---|---|---|---|
| **casecntl** | **Intensity(Intensity) x 10** | | | |
| **Frequency Row Pct Col Pct** | **<=2000** | **2001-4000** | **>=4001** | **Total** |
| **control** | 27 | 74 | 1741 | 1842 |
| | 1.47 | 4.02 | 94.52 | |
| | 52.94 | 43.53 | 67.04 | |
| **patient** | 24 | 96 | 856 | 976 |
| | 2.46 | 9.84 | 87.70 | |
| | 47.06 | 56.47 | 32.96 | |
| **Total** | 51 | 170 | 2597 | 2818 |

**Statistics for Table 4 of casecntl by Intensity**

| **Statistic** | **DF** | **Value** | **Prob** |
|---|---|---|---|
| **Chi-Square** | 2 | 42.4945 | <.0001 |
| **Likelihood Ratio Chi-Square** | 2 | 40.2604 | <.0001 |
| **Mantel-Haenszel Chi-Square** | 1 | 27.0821 | <.0001 |
| **Phi Coefficient** | | 0.1228 | |
| **Contingency Coefficient** | | 0.1219 | |
| **Cra'er's V** | | 0.1228 | |

Table 5: Moderate AD - sample size: 2046

| Table of casecntl by Intensity | | | | |
|---|---|---|---|---|
| **casecntl** | **Intensity(Intensity) x 10** | | | |
| **Frenquency Row Pct Col Pct** | **<= 2000** | **2001-4000** | **>=4001** | **Total** |
| **control** | 2 | 6 | 1247 | 1255 |
| | 0.16 | 0.48 | 99.36 | |
| | 7.69 | 8.45 | 63.98 | |
| **patient** | 24 | 65 | 702 | 791 |
| | 3.03 | 8.22 | 88.75 | |
| | 92.31 | 91.55 | 36.02 | |
| **Total** | 26 | 71 | 1949 | 2046 |

**Statistics for Table 5 of casecntl by Intensity**

Table 6: Advanced AD - sample size: 1738

| **Statistic** | **DF** | **Value** | **Prob** |
|---|---|---|---|
| **Chi-Square** | 2 | 121.0396 | <.000 1 |
| **Likelihood Ratio Chi-Square** | 2 | 127.5404 | <.000 1 |
| **Mantel-Haenszel Chi-Square** | 1 | 98.4856 | <.000 1 |

(continued)

| Statistic | DF | Value | Prob |
|---|---|---|---|
| Phi Coefficient | | 0.2432 | |
| Contingency Coefficient | | 0.2363 | |
| Cra'er's V | | 0.2432 | |

| Table of casecntl by Intensity | | | | |
|---|---|---|---|---|
| casecntl | Intensity(Intensity) x 10 | | | |
| Frequency Row Pct Col Pct | <=2000 | 2001-4000 | >=4001 | Total |
| control | 43 | 84 | 988 | 1115 |
| | 3.86 | 7.53 | 88.61 | |
| | 81.13 | 66.67 | 63.37 | |
| patient | 10 | 42 | 571 | 623 |
| | 1.61 | 6.74 | 91.65 | |
| | 18.87 | 33.33 | 36.63 | |
| Total | 53 | 126 | 1559 | 1738 |

**Statistics for Table 6 of casecntl by Intensity**

| Statistic | DF | Value | Prob |
|---|---|---|---|
| Chi-Square | 2 | 7.4018 | 0.0247 |
| Likelihood Ratio Chi-Square | 2 | 8.1176 | 0.0173 |
| Mantel-Haenszel Chi-Square | 1 | 6.8334 | 0.0089 |
| Phi Coefficient | | 0.0653 | |
| Contingency Coefficient | | 0.0651 | |
| Cra'er's V | | 0.0653 | |

## Example 4

Telomeres get progressively shorter with each cell division. Changes in telomere length are associated with the process of aging and some age related syndromes. Whether the three-dimensional (3D) nuclear organization of telomeres is altered in Alzheimer's disease (AD) and during the initiation and progression of this disease was investigated. To this end, buccal swaps of AD patients and age-matched controls were utilized. Buccal cells (BCs) were examined after 3D Q-FISH of telomeres, 3D imaging of telomeres and quantitative analysis using TeloView software. Fifty-nine patients with Alzheimer's disease (AD) (ranging in stages from mild to severe) and their fifty-nine cognitively normal age-matched caregivers were included in the study. Significantly higher numbers of telomeres with lower intensity signals and decreased nuclear volumes in AD patients and during disease initiation and progression were found compared to controls. The data suggest significant differences in nuclear architecture of BCs in AD and normal age-matched controls.

Fifty-nine patients with AD (ranging in stage from mild to severe) and their fifty-nine cognitively normal age-matched caregivers were included in this study. Buccal cells (BCs) were used in the study because they many advantages; not only can the samples be collected non-invasively, but BCs also originate from the neuro-ectoderm, which is where brain tissue is derived from. A previous study had shown that BCs from AD patients had significantly shorter telomere length than age-matched healthy controls [Thomas P et al, (2008). However, the authors looked only at telomere length using PCR technique and did not investigate the 3D nuclear telomere organization in AD. Using Q-FISH to localize the telomeres in the nucleus of BC samples and analyzing them by TeloView [Vermolen BJ et al, 2005] the 3D architecture of telomeres in patients with AD and aged-matched controls has been investigated.

**Population and Methods:**

Subjects: In the study, fifty-nine patients with Alzheimer's Disease (AD) attending the Queen's University Memory Clinics were compared to their fifty-nine age-matched (+/- 5 years) cognitively normal caregivers according to the following parameters: telomere aggregation, telomere length, a/c ratio, nuclear volume and Fisher's exact test for telomere numbers. Diagnosis of AD was made according to the National Institute of Neurological and Communicative Disorders and Stroke, and the Alzheimer's Disease and Related Disorders Association (NINCDS-ADRDA) Alzheimer's criteria and all patients had been on standard treatment according to the Canadian Guidelines for the Treatment of Dementia. Patients were followed at the Memory Clinics and the dementia was staged as mild, moderate or severe, according to their scores in the Montreal Cognitive Assessment test (MoCA) and/or the Mini-Mental State Exam (MMSE). Patients with mild AD (n=31) had Montreal Cognitive Assessment (MoCA) scores between 24/30 and 18/30. Patients with moderate AD (n=12) had MMSE scores between 16/30 and 21/30 inclusive and patients with advanced AD (n=16) had MMSE scores <16/30.

Buccal swabs

Buccal swabs were obtained in duplicate, at the regular clinic visits, using sterile Epicenter Biotechnologies swabs. Samples from each swab were smeared on VWR micro slides and kept frozen at -20° C for 1 to 3 months until Q-FISH analysis.

3D quantitative fluorescent in situ hybridization (3D Q-FISH)

Cells were fixed with 3.7% formaldehyde (Fisher). Hybridization was completed using a DAKO Cy3 telomere PNA probe (DAKO, Glostrup, Denmark). Three-dimensional telomere quantitative FISH (3D-Q-FISH) was performed as described previously [Knecht H et al., 2009; Knecht H et al., 2010].

Quantitative image Analysis

Digital images were taken using Zeiss AxioImager Z1 with a cooled AxioCam HR B&W, DAPI, Cy3 filters in combination with a Planapo 63x/1.4 oil objective lens. Images were acquired by using AXIOVISION 4.8 (Zeiss) in multichannel mode followed by constrained iterative deconvolution [Schaefer LH et al., (2010)]. For every fluorochrome, 80 images stacks were acquired with a sampling distance of 200 nm along the z and 102 nm in the xy direction. Thirty interphase nuclei from each AD sample, as well as a healthy control, were analyzed.

3D Telomere Analysis

Quantification of the 3D nuclear telomeric signals was performed using TeloView [Vermolen BJ et al., (2005)]. After finding the threshold for the telomeres, a binary image opens and the volume, intensity and center of gravity are calculated. The program analyzed the center of intensities for every object resulting in a set of coordinates (x, y, z). For each telomere that is proportional in length the integrated intensity of each is calculated [Poon SS, et al., (1999)].

Telomere aggregates

Telomere aggregates are clusters of telomeres that are in close association to each other and cannot be further resolved as separate entities because of an optical resolution limit of 200 nm [Mai S and Garini Y, (2006)]

Telomere length

Telomeres were classified depending on the relative fluorescent intensity (x-axis) as: Short: 0-20000 units, Mid-size: 20001-40000 units and large: >40001 units. These ranges were proposed by looking at the profiles and were over 95% accurate in categorizing the disease stages according to their diagnosis.

A/c ratio and the nuclear volume

The nuclear space occupied by telomeres is measured by three axes, a, b and c where a, b, are equal in length, and c, has a different length. The distribution of telomeres in the three-dimensional space of the nucleus varies with cell cycle; as the specific stages of the cell cycle (G0/G1, S, and G2) phases have characteristic a/c ratios, therefore, the investigator can determine where they reside in the cell cycle. The a/c ratio allows defining progression through cell cycle in interphase cells [Vermolen BJ et al., (2005)].

Nuclear volume is calculated according to the 3D nuclear 4', 6-diamidino-2-phenylindole staining (DAPI) [Vermolen BJ et al., (2005)].

Statistics

3D-Q-FISH results were analyzed by Fisher's exact test for telomere numbers. Chi-square was used to determine telomere length and differences between the AD groups and healthy age matched controls. A P value <0.05 was considered statistically significant.

**Results**

Increased number and decreased telomere length in BCs from AD patients.

First the total number and length of telomeres were analysed in each AD group and healthy controls using Teloview [Vermolen BJ et al., (2005)]. Similar results to Figure 1 were found. The results show that AD patients have an increasing number of telomeres as their disease progresses to a more severe stage, with the advanced stage showing the highest numbers of telomeres. The age-matched controls however, tend to remain within the normal range (40-60 telomeres, de Vos et al., 2009) regardless of the control's age or gender. AD patients also show a decrease in telomeres length as their disease progresses. Telomere nuclear signals for each patient category in two-dimensional and three-dimensional images as determined using imaging and three-dimensional reconstruction after constrained iterative deconvolution. The 2D images found interphase nuclei with higher numbers of telomeres in patients with AD as compared to age-matched controls. The 3D images show increased telomeric signals as well with a decrease in length of telomeres in AD as compared to controls samples.

Telomere Aggregates (TAS) are not altered in AD.

Using the Teloview software, the number of telomeric aggregation (TAS) in the three AD groups was analyzed and compared to healthy controls. No significant changes in numbers of TAS in AD patients compared to controls (Mild p=0.1246, Moderate p=0.0519 and Advanced p=0.1171) were found.

Decreased nuclear volume of ADs samples is not related to changes in proliferations in BCs

Using the 3D imaging software, the cell morphology of the Buccal Cells (BCs) was studied by analyzing their a/c ratios and nuclear volume. The results show a statistically significant decreased nuclear volume of BCs in all three groups of AD compared to controls (Mild p<0.0001, Moderate p<0.0005 and Advanced p<0.0001) was observed (Tables 7-10). To determinate if decreased in nuclear volume is related to cells proliferation the a/c ratio in BCs cells was assessed. No significant alteration in a/c ratio in all three groups of AD as compare to ached-matched controls (Mild p=0.11, Moderate p=0.74 and Advanced p=0.74) was detected suggesting that smaller nuclear volume of BCs in ADs is not related to cell cycle.

**Table 7: Nuclear Volume Statistics for Mild AD Representative Graph**

| Wilcoxon Scores (Rank Sums) for Variable Nuclear volume Classified by Variable casecntl | | | | | |
|---|---|---|---|---|---|
| casecntl | N | Sum of Scores | Expected Under H0 | Std Dev Under H0 | Mean Score |
| control | 30 | 1151.0 | 915.0 | 67.638746 | 38.366667 |
| patient | 30 | 679.0 | 915.0 | 67.638746 | 22.633333 |
| Wilcoxon Two-Sample Test | | | | | |
| Statistic | | | | | 1151.0000 |
| | | | | | |
| Normal | | | | | |
| Z | | | | | 3.4817 |

(continued)

| Normal | |
|---|---|
| One-Sided Pr > Z | 0.0002 |
| Two-Sided Pr > \|Z\| | 0.0005 |
| | |
| t Approximation | |
| One-Sided Pr > Z | 0.0005 |
| Two-Sided Pr > \|Z\| | 0.0009 |
| Z includes a continuity correction of 0.5. | |
| **Kruskal-Wallis Test** | |
| Chi-Square | 12.1740 |
| DF | 1 |
| Pr > Chi-Square | 0.0005 |

**Table 8: Nuclear Volume Statistics for Moderate AD Representative Graph**

| Wilcoxon Scores (Rank Sums) for Variable Nuclear volume Classified by Variable casecntl | | | | | |
|---|---|---|---|---|---|
| casecntl | N | Sum of Scores | Expected Under H0 | Std Dev Under H0 | Mean Score |
| control | 30 | 1117.0 | 915.0 | 67.638746 | 37.233333 |
| patient | 30 | 713.0 | 915.0 | 67.638746 | 23.766667 |
| **Wilcoxon Two-Sample Test** | | | | | |
| Statistic | | | | | 1117.0000 |
| | | | | | |
| Normal Approximation | | | | | |
| Z | | | | | 2.9791 |
| One-Sided Pr > Z | | | | | 0.0014 |
| Two-Sided Pr > \|Z\| | | | | | 0.0029 |
| | | | | | |
| t Approximation | | | | | |
| One-Sided Pr > Z | | | | | 0.0021 |
| Two-Sided Pr > \|Z\| | | | | | 0.0042 |
| Z includes a continuity correction of 0.5. | | | | | |
| **Kruskal-Wallis Test** | | | | | |
| Chi-Square | | | | | 8.9189 |
| DF | | | | | 1 |
| Pr > Chi-Square | | | | | 0.0028 |

**Table 9: Nuclear Volume Statistics for Advanced AD Representative Graph**

| Wilcoxon Scores (Rank Sums) for Variable Nuclear volume Classified by Variable casecntl | | | | | |
|---|---|---|---|---|---|
| casecntl | N | Sum of Scores | Expected Under H0 | Std Dev Under H0 | Mean Score |
| control | 30 | 1349.0 | 915.0 | 67.638746 | 44.966667 |
| patient | 30 | 481.0 | 915.0 | 67.638746 | 16.033333 |
| Wilcoxon Two-Sample Test | | | | | |
| Statistic | | | | | 1349.0000 |
| | | | | | |
| Normal Approximation | | | | | |
| Z | | | | | 6.4090 |
| One-Sided Pr > Z | | | | | <.0001 |
| Two-Sided Pr > |Z| | | | | | <.0001 |
| | | | | | |
| t Approximation | | | | | |
| One-Sided Pr > Z | | | | | <.0001 |
| Two-Sided Pr > |Z| | | | | | <.0001 |
| Z includes a continuity correction of 0.5. | | | | | |
| Kruskal-Wallis Test | | | | | |
| Chi-Square | | | | | 41.1707 |
| DF | | | | | 1 |
| Pr > Chi-Square | | | | | <.0001 |

**Table 10: Five randomly chosen representative statistics on a/c ratio and nuclear volume (The patients were selected with disregard to their AD disease stage)**

| Level of casecntl | Level of unit | N | acratio | | Nuclear volume | |
|---|---|---|---|---|---|---|
| | | | Mean | Std Dev | Mean | Std Dev |
| control | 29 | 31 | 5.0883402 | 1.6112826 | 542312.67 | 94630.770 |
| patient | 29 | 33 | 5.2148131 | 2.9345860 | 368785.13 | 338519.103 |
| control | 33 | 30 | 6.7835816 | 2.8685095 | 496497.77 | 190697.949 |
| patient | 33 | 32 | 4.6374265 | 1.6151638 | 357084.78 | 123871.077 |
| control | 45 | 30 | 5.5982671 | 1.6308646 | 563854.00 | 138114.830 |
| patient | 45 | 30 | 4.5170006 | 1.8195579 | 435992.00 | 150009.229 |
| control | 51 | 30 | 9.0832502 | 3.8431860 | 1067085.4 0 | 279181.790 |
| patient | 51 | 30 | 5.3289436 | 2.6183706 | 1075780.5 0 | 217608.838 |
| control | 54 | 30 | 10.7643725 | 5.9442943 | 1254834.6 3 | 393183.852 |
| patient | 54 | 30 | 7.8002197 | 2.7682179 | 927393.53 | 270204.209 |

All AD patients except for one showed a 10-50% decrease in their nuclear volumes as compared to their control.

**Discussion**

The aim of the study was to investigate the 3D nuclear organization of telomeres obtained from BCs of Alzheimer patients and compare them to BCs of healthy age-matched controls. Q-FISH was used to localize the telomeres in the nucleus of our samples. The results show significant higher numbers of telomeres and shorter telomere length in AD patients at any stage of the disease, when compared to healthy age matched controls. A decrease in nuclear volume in AD patients compared to controls was also seen.

This is the first study to relate changes in 3D nuclear architecture from BCs to the mental status/stage of AD patients. A study by Panossian et al [Panossian LA et al., (2003)] investigating AD patients' mental status and telomeres looked only at telomere length in T-cells using Telomere length analysis. Within the AD group they observed a significant correlation between telomere length of T cells and MMSE scores. Using the 3D approach and BCs, not only telomere length but also telomere numbers was analyzed in relation to AD stages, to investigate the presence of aggregates, the cell cycle distribution and nuclear volumes in AD vs. controls. BCs have been used in one previous Alzheimer study only to look at telomere length in different age groups of AD patients to compare them to BCs from healthy age-related controls [Thomas P et al, (2008)] as well as to other cells from the same populations.

Significantly shorter telomere lengths were found in BCs in all three groups of AD patients compared to their age-matched healthy controls (Table 1). It is still unknown, however, why people with AD have shorter telomere length. Telomeres shorten with each cell division because of an end replication problem. Loss of telomeric DNA activates a p53-dependent checkpoint that leads to apoptosis or senescence (Milyavsky M et al., (2001); Farazi PA et al., (2006)). Telomerase solves this problem by synthesizing new telomeres. The accumulation of the damaged DNA bases in cells may result in the loss of normal cellular function, which may be contributing to AD and other age-related diseases [Myung NH et al., (2008), Coppede F and Migliore L (2009)]. Many proteins related to DNA damage are altered in AD. The Mre11 protein complex consisting of Rad50, Mre11 and Nbs1 is essential for cellular responses to DNA damage, such as initiating cell cycle checkpoints and repairing damaged DNA [Mirzoeva OK and Petrini JH (2001); Delmas S et al. (2009)]. The Mre11 complex is present in adult human cortex and cerebellum neurons. This complex is reduced in the cortical neurons of patients with AD. The accumulated DNA damage in AD neurons may be, in part, the result of the reduced levels of Mre11 protein complexes [Jacobson SJ et al, (2004)]. Repair of doublestranded breaks requires a DNA-dependent protein kinase, which is composed of DNA-PKcs and Ku. Ku DNA binding activity is reduced in extracts of postmortem AD mid-frontal cortex [Davydov V, et al., (2003)]. The decreased Ku DNA binding is positively correlated with reduced protein levels of Ku subunits (DNA-PKcs) and poly (ADP-ribose) polymerase-1.

To date, most AD research has been done on brain cells, not on buccal cells [Davydov V, Hansen LA, Shackelford DA (2003)] BCs' DNA repair capacity is limited in comparison to the peripheral blood cells and lymphocytes. It has been shown that BCs have shorter telomeres in AD than in controls [Thomas P, et al, (2008)]. BCs reflect the actual age-related changes in genomic instability in epithelial cells [Dhillon VS, et al. (2004); Carlin V et al. 2011; Thomas P, et al. (2008)].

The studies using 3D nuclear imaging also revealed higher numbers of telomeres in cells of AD patients. Dysfunction of telomeres leads to the arrest of the cell cycle and initiates cell apoptosis or cell senescence [Coppede F et al., (2009); Lechel A el al. 2005; Herbig U et al. 2006]. Neuronal cell death is a pathological hallmark of AD. Apoptosis and other alternative pathways of neuronal cell death have also been investigated in AD. It is still unclear how changes in telomere signals contribute to cell death in AD and what mechanisms contribute to these alterations. Telomere maintenance depends heavily on telomere binding proteins, of which telomere repeat binding factor 2 (TRF2) is a critical member. TRF2 is one of six proteins consisting of the protein complex shelterin capping the telomeres [de Lange T. (2005)]. Shelterin is involved in telomere length regulation and telomere structure maintenance [de Lange T. (2005)]. Loss or mutation of TRF2 is associated with telomeric structure destruction, DNA damage, apoptosis and senescence [Lechel A el al. 2005, an Steensel B, et al 1998, Smogorzewska A, 2002]. Therefore, further research on TRF2 and shelterins is needed to understand telomere maintenance in AD.

Nuclear volume of the BCs in all three groups of ADs patients was analysed and compared to age-matched controls. Significant decreases were observed in nuclear volume in ADs. The size of the cells is changing during the cell cycle although no changes in a/c ratio were found suggesting that smaller nuclear volume of BCs in ADs is not related to cell cycle [Umen JG (2005; Echave Pet al (2007)]. It has been shown that size of the nucleus is also related to chromatin remodeling and involves lamin A [Broers JL,et al (2006)]. A-type lamin is an important protein influencing nuclear architecture by providing the scaffolds for the organization of nuclear function [Taddei A, et al, (2004); Dechat T, et al, (2008)]. Loss of lamin A contributes to pathogenesis of lamin-related diseases, especially premature aging syndromes, such as Hutchinson-Gilford progeria syndrome (HGPS) [Huang S et al, (2008); Mounkes LC, Stewart CL (2004); Prokocimer M, et al., (2009)]. Studies showed that knockout of lamin A leads to variety of changes in telomere biology including: i) nuclear decompartmentalization of telomeres; ii) impairment of telomere length and iii) defects in telomere chromatin architecture [Raz V, et al (2008)]. Using the TeloView, the presence or absence of telomeric aggregates (TAs) a/c ratio and nuclear volume was determined in our samples. TAs are defined as clusters of telomeres found in close proximity to each other and that cannot be resolved as separate entities because of the optical resolution limit of 200 nm. There were no significant differences in numbers of TAs in AD groups as compared to aged-matched healthy controls. TAs are aberrations in the nuclear organization seen in tumor cells [Mai S, Garini Y(2006); Mai S, Garini Y (2005); Gadji M et al (2010)]. When TAs form and chromosome fusions occur, breakage/bridge/fusion (BBF) cycles result. This then remodels the genetic information of the chromosomes [Louis SF, et al (2005); Guffei A, et al. (2010)]. TA formation is independent of telomere size and telomerase activity [Louis SF, et al (2005)]. Lack of differences in TA formations in ADs and healthy age-matched controls suggests that i) nuclear remodeling in AD is different from that found in tumors, and ii) clusters of telomeres are not the reason for the lower numbers of telomeres in AD samples.

In conclusion, the study describes the detailed nuclear telomeric architecture of BCs in AD and compares it to healthy age-matched controls. A significantly higher number of telomeres and shorter telomere signals in three different groups of AD patients were found. TA formations were not observed in AD or healthy controls. 3D-Q-FISH analysis is an excellent discriminator for the identification of changes in the nuclear telomeric architecture of AD. The 3D telomeric signatures identified in AD are associated with all disease stages; mild, moderate and severe.

**Table 11: Population characteristics**

| Population | Number of Subjects | Mean age | Sex | AD stage | Mean Score MoCA - MMSE |
|---|---|---|---|---|---|
| AD mild | 31 | 72.57 | 7M/6F | MILD | 22.8 23.5 |
| Control | 31 | 69.14 | 9F/4M | Normal | |
| AD moderate | 12 | 77.5 | 3F | MOD. | 20 |
| Control | 12 | 77.5 | 3M | Normal | |
| AD advanced | 16 | 70.0 | 2F/2M | ADV | 7 |
| Control | 16 | 69.0 | 2M/2F | Normal. | |

AD = Alzheimer's disease; M = male; F = female; MOD = Moderate AD; ADV = Advanced AD; MoCA = Montreal Cognitive Assessment test score; MMSE = Mini-Mental State Examination test score. ----- = No MoCA score. The numbers are the mean scores for the AD groups. Only the mild group has a MoCA score, the moderate and advanced only have an MMSE score.

## CITATIONS

Blackburn, E. H. (1991) Nature 350, 569-573

Greider, C. W. (1996) Annu Rev Biochem 65, 337-365

Blackburn, E. H. (1992) Annu Rev Biochem 61, 113-129

Buchkovich, K. J., and Greider, C. W. (1996) Mol Biol Cell 7, 1443-1454

Slagboom, P. E., Droog, S., and Boomsma, D. I. (1994) Am J Hum Genet 55, 876-882

Rudolph, K. L., Chang, S., Lee, H. W., Blasco, M., Gottlieb, G. J., Greider, C., and DePinho, R. A. (1999) Cell 96, 701-712

Chan, S. W., and Blackburn, E. H. (2002) Oncogene 21, 553-563

Fry, M., and Loeb, L. A. (1999) J Biol Chem 274, 12797-12802

Shen, J. C., Gray, M. D., Oshima, J., Kamath-Loeb, A. S., Fry, M., and Loeb, L. A. (1998) J Biol Chem 273, 34139-34144

Yu, C. E., Oshima, J., Fu, Y. H., Wijsman, E. M., Hisama, F., Alisch, R., Matthews, S., Nakura, J., Miki, T., Ouais, S., Martin, G. M., Mulligan, J., and Schellenberg, G. D. (1996) Science 272, 258-262

Hayflick, L. (1977) Handbook of the bilology aging, 20

Sohal, R. S., and Allen, R. G. (1985) Basic Life Sci 35, 75-104

Vulliamy, T. J., Knight, S. W., Mason, P. J., and Dokal, I. (2001) Blood Cells Mol Dis 27, 353-357

Charames, G. S., and Bapat, B. (2003) Curr Mol Med 3, 589-596

Holland, A. J., and Cleveland, D. W. (2009) Nat Rev Mol Cell Biol 10, 478-487

Knecht, H., Bruderlein, S., Wegener, S., Lichtensztejn, D., Lichtensztejn, Z., Lemieux, B., Moller, P., and Mai, S. BMC Cell Biol 11, 99

Lothe, R. A., Peltomaki, P., Meling, G. I., Aaltonen, L. A., Nystrom-Lahti, M., Pylkkanen, L., Heimdal, K., Andersen, T. I., Moller, P., Rognum, T. O., and et al. (1993) Cancer Res 53, 5849-5852

Colleu-Durel S, G. N., Nourgalieva K, Lévêque J, Danic B, Chenal C. (2001) Oncol Rep 8, 4

Guffei, A., Sarkar, R., Klewes, L., Righolt, C., Knecht, H., and Mai, S. Haematologica 95, 2038-2046

de Lange, T. (2002) Oncogene 21, 532-540

Masutomi, K., Yu, E. Y., Khurts, S., Ben-Porath, I., Currier, J. L., Metz, G. B., Brooks, M. W., Kaneko, S., Murakami, S., DeCaprio, J. A., Weinberg, R. A., Stewart, S. A., and Hahn, W. C. (2003) Cell 114, 241-253

Harley, C. B., Futcher, A. B., and Greider, C. W. (1990) Nature 345, 458-460

Huffman, K. E., Levene, S. D., Tesmer, V. M., Shay, J. W., and Wright, W. E. (2000) J Biol Chem 275, 19719-19722

Kirwan, M., and Dokal, I. (2009) Biochim Biophys Acta 1792, 371-379

Chang, S., Multani, A. S., Cabrera, N. G., Naylor, M. L., Laud, P., Lombard, D., Pathak, S., Guarente, L., and DePinho, R. A. (2004) Nat Genet 36, 877-882

Thomas, P., NJ, O. C., and Fenech, M. (2008) Mech Ageing Dev 129, 183-190

Thomas, P., Hecker, J., Faunt, J., and Fenech, M. (2007) Mutagenesis 22, 371-379

Panossian, L. A., Porter, V. R., Valenzuela, H. F., Zhu, X., Reback, E., Masterman, D., Cummings, J. L., and Effros, R. B. (2003) Neurobiol Aging 24, 77-84

Burns, A., Byrne, E. J., and Maurer, K. (2002) Lancet 360, 163-165

Du, A. T., Schuff, N., Amend, D., Laakso, M. P., Hsu, Y. Y., Jagust, W. J., Yaffe, K., Kramer, J. H., Reed, B., Norman, D., Chui, H. C., and Weiner, M. W. (2001) J Neurol Neurosurg Psychiatry 71, 441-447

Haroutunian, V., Perl, D. P., Purohit, D. P., Marin, D., Khan, K., Lantz, M., Davis, K. L., and Mohs, R. C. (1998) Arch Neurol 55, 1185-1191

Kawas, C. H. (2003) N Engl J Med 349, 1056-1063

Mattson, M. P. (2004) Nature 430, 631-639

Iqbal, K., Grundke-Iqbal, I., Smith, A. J., George, L., Tung, Y. C., and Zaidi, T. (1989) Proc Natl Acad Sci U S A 86, 5646-5650

Hunt, A. J., and McIntosh, J. R. (1998) Mol Biol Cell 9, 2857-2871

Iqbal, K., Alonso, A. C., Gong, C. X., Khatoon, S., Pei, J. J., Wang, J. Z., and Grundke-Iqbal, I. (1998) J Neural Transm Suppl 53, 169-180

Petkova, A. T., Ishii, Y., Balbach, J. J., Antzutkin, O. N., Leapman, R. D., Delaglio, F., and Tycko, R. (2002) Proc Natl Acad Sci U S A 99, 16742-16747

Antzutkin, O. N., Leapman, R. D., Balbach, J. J., and Tycko, R. (2002) Biochemistry 41, 15436-15450

Thomas, P., and Fenech, M. (2008) Mutagenesis 23, 57-65

Nasreddine, Z. S., Phillips, N. A., Bedirian, V., Charbonneau, S., Whitehead, V., Collin, I., Cummings, J. L., and Chertkow, H. (2005) J Am Geriatr Soc 53, 695-699

Folstein, M. F., Folstein, S. E., and McHugh, P. R. (1975) J Psychiatr Res 12, 189-198

McKhann, G., Drachman, D., Folstein, M., Katzman, R., Price, D., and Stadlan, E. M. (1984) Neurology 34, 939-944

Patterson, C. J., Gauthier, S., Bergman, H., Cohen, C. A., Feightner, J. W., Feldman, H., and Hogan, D. B. (1999) CMAJ 160, 1738-1742

Chuang, T. C., Moshir, S., Garini, Y., Chuang, A. Y., Young, I. T., Vermolen, B., van den Doel, R., Mougey, V., Perrin, M., Braun, M., Kerr, P. D., Fest, T., Boukamp, P., and Mai, S. (2004) BMC Biol 2, 12

Louis, S. F., Vermolen, B. J., Garini, Y., Young, I. T., Guffei, A., Lichtensztejn, Z., Kuttler, F., Chuang, T. C., Moshir, S., Mougey, V., Chuang, A. Y., Kerr, P. D., Fest, T., Boukamp, P., and Mai, S. (2005) Proc Natl Acad Sci U S A 102, 9613-9618

Klewes, L., Höbsch, C., Katzir, N., Rourke, D., Garini, Y., and Mai, S. (2011) Cytometry Part A 79A, 159-166.47. Knecht H, Sawan B, Lichtensztejn Z, Lichtensztejn D, Mai S. Lab Invest. 2010;90(4):611-619).

Blackburn EH (1991) Structure and function of telomeres. Nature 350, 569-73. de Lange T (2002) Protection of mammalian telomeres. Oncogene 21, 532-40.

Harley CB, Futcher AB, Greider CW (1990) Telomeres shorten during ageing of human fibroblasts. Nature 345, 458-60.

Huffman KE, Levene SD, Tesmer VM, Shay JW, Wright WE. (2000) Telomere shortening is proportional to the size of the G-rich telomeric 3'-overhang. J Biol Chem 275, 19719-22.

Charames GS, Bapat B (2003) Genomic instability and cancer. Curr Mol Med 3, 589-96.

Holland AJ, Cleveland DW (2009) Boveri revisited: chromosomal instability, aneuploidy and tumorigenesis. Nat Rev Mol Cell Biol 10, 478-87.

Lothe RA, Peltomaki P, Meling GI (1993) Genomic instability in colorectal cancer: relationship to clinicopathological variables and family history. Cancer Res 53, 5849-52.

Colleu-Durel S, Guitton N, Nourgalieva K, Leveque J, Danic B, Chenal C (2001) Genomic instability and breast cancer. Oncol Rep 8, 1001-5.

Chan SW, Blackburn EH (2002) New ways not to make ends meet: telomerase, DNA damage proteins and heterochromatin. Oncogene 21, 553-63.

Hayflick, L (1979) The Cell Biology of Aging. Journal of Investigative Dermatology 73, 8-14.

Sohal RS, Allen RG (1985) Relationship between metabolic rate, free radicals, differentiation and aging: a unified theory. Basic Life Sci 35, 75-104.

Yu CE, Oshima J, Fu YH (1996) Positional cloning of the Werner's syndrome gene. Science 272, 258-62.

Shen JC, Gray MD, Oshima J, Kamath-Loeb AS, Fry M, Loeb LA (1998). Werner syndrome protein. I. DNA helicase and dna exonuclease reside on the same polypeptide. J Biol Chem 273, 34139-44.

Fry M, Loeb LA (1999). Human werner syndrome DNA helicase unwinds tetrahelical structures of the fragile X syndrome repeat sequence d(CGG)n. J Biol Chem 274, 12797-802.

Burns A, Byrne EJ, Maurer K (2002) Alzheimer's disease. Lancet 360, 163-5.

Panossian LA, Porter VR, Valenzuela HF (2003) Telomere shortening in T cells correlates with Alzheimer's disease status. Neurobiol Aging 24, 77-84.

Thomas P, Hecker J, Faunt J, Fenech M (2007) Buccal micronucleus cytome biomarkers may be associated with Alzheimer's disease. Mutagenesis 22, 371-9.

Du AT, Schuff N, Amend D (2001) Magnetic resonance imaging of the entorhinal cortex and hippocampus in mild cognitive impairment and Alzheimer's disease. J Neurol Neurosurg Psychiatry 71, 441-7.

Haroutunian V, Perl DP, Purohit DP (1998) Regional distribution of neuritic plaques in the nondemented elderly and subjects with very mild Alzheimer disease. Arch Neurol 55, 1185-91.

Mattson MP (2004) Pathways towards and away from Alzheimer's disease. Nature 430, 631-9.

Kawas CH (2003) Clinical practice. Early Alzheimer's disease. N Engl J Med 349, 1056-63.

Iqbal K, Alonso AC, Gong CX(1998) Mechanisms of neurofibrillary degeneration and the formation of neurofibrillary tangles J Neural Transm Suppl 53, 169-80.

Petkova AT, Ishii Y, Balbach JJ (2002) A structural model for Alzheimer's beta - amyloid fibrils based on experimental constraints from solid state NMR. Proc Natl Acad Sci U S A 99, 16742-7.

Antzutkin ON, Leapman RD, Balbach JJ, Tycko R (2002) Supramolecular structural constraints on Alzheimer's beta-amyloid fibrils from electron microscopy and solid-state nuclear magnetic resonance. Biochemistry 41, 15436-50.

Thomas P, NJ OC, Fenech M (2008) Telomere length in white blood cells, buccal cells and brain tissue and its variation with ageing and Alzheimer's disease. Mech Ageing Dev 129, 183-90.

Cawthon RM, Smith KR, O'Brien E, Sivatchenko A, Kerber RA (2003) Association between telomere length in blood and mortality in people aged 60 years or older. Lancet 361, 393-5.

Honig SL, Tang MX, Albert S, Costa R (2003) Stroke and the Risk of Alzheimer Disease.Archives of Neurology 60, 1707-1712.

Lukens JN, Van Deerlin V, Clark CM, Xie SX, Johnson FB (2009) Comparisons of telomere lengths in peripheral blood and cerebellum in Alzheimer's disease. Alzheimers Dement 5, 463-9.

Vermolen BJ, Garini Y, Mai S (2005) Characterizing the three-dimensional organization of telomeres. Cytometry A 67, 144-50.

McKhann G, Drachman D, Folstein M, Katzman R, Price D, Stadlan EM (1984) Clinical diagnosis of Alzheimer's disease: report of the NINCDS-ADRDA Work Group under the auspices of Department of Health and Human Services Task Force on Alzheimer's Disease. Neurology 34, 939-44.

Hogan DB, Bailey P, Black S. Diagnosis and treatment of dementia: 5. (2008) Nonpharmacologic and pharmacologic therapy for mild to moderate dementia. CMAJ 176, 1019-1026.

Nasreddine ZS, Phillips NA, Bedirian V (2005) The Montreal Cognitive Assessment, MoCA: a brief screening tool for mild cognitive impairment. J Am Geriatr Soc 53, 695-9.

Folstein MF, Folstein SE, McHugh PR (1975)"Mini-mental state". A practical method for grading the cognitive state of patients for the clinician. J Psychiatr Res 12, 189-98.

Knecht H, Sawan B, Lichtensztejn D, Lemieux B, Wellinger RJ, Mai S. (2009) The 3D nuclear organization of telomeres marks the transition from Hodgkin to Reed-Sternberg cells. Leukemia 23, 565-73.

Knecht H, Bruderlein S, Wegener S, Mai S (2010) 3D nuclear organization of telomeres in the Hodgkin cell lines U-HO1 and U-HO1-PTPN1: PTPN1 expression prevents the formation of very short telomeres including "t-stumps". BMC Cell Biol 11, 99-102

Schaefer LH, Schuster D, Herz H (2010) Generalized approach for accelerated maximum likelihood based image restoration applied to three-dimensional fluorescence microscopy. J Microsc 204, 99-107.

Poon SS, Martens UM, Ward RK, Lansdorp PM (1999) Telomere length measurements using digital fluorescence microscopy. Cytometry 36, 267-78.

Mai S, Garini Y(2006) The significance of telomeric aggregates in the interphase nuclei of tumor cells. J Cell Biochem 97, 904-15.

Milyavsky M, Mimran A, Senderovich S (2001) Activation of p53 protein by telomeric (TTAGGG)n repeats. Nucleic Acids Res 29, 5207-15.

Farazi PA, Glickman J, Horner J, Depinho RA (2006) Cooperative interactions of p53 mutation, telomere dysfunction, and chronic liver damage in hepatocellular carcinoma progression. Cancer Res 66, 4766-73.

Myung NH, Zhu X, Kruman, II (2008) Evidence of DNA damage in Alzheimer disease: phosphorylation of histone H2AX in astrocytes. Age (Dordr) 30, 209-15.

Coppede F, Migliore L (2009) DNA damage and repair in Alzheimer's disease. Curr Alzheimer Res 6, 36-47.

Mirzoeva OK, Petrini JH (2001) DNA damage-dependent nuclear dynamics of the Mre11 complex. Mol Cell Biol 21, 281-8.

Delmas S, Shunburne L, Ngo HP, Allers T (2009) Mre11-Rad50 promotes rapid repair of DNA damage in the polyploid archaeon Haloferax volcanii by restraining homologous recombination. PLoS Genet 5, e1000552.

Jacobson SJ, Laurenson PM, Pillus L (2004) Functional analyses of chromatin modifications in yeast. Methods Enzymol 377, 3-55.

Davydov V, Hansen LA, Shackelford DA (2003) Is DNA repair compromised in Alzheimer's disease? Neurobiol Aging 24, 953-68.

Davydov V, Hansen LA, Shackelford DA (2003) Is DNA repair compromised in Alzheimer's disease? Neurobiol Aging 24, 953-68.

Shackelford DA (2006) DNA end joining activity is reduced in Alzheimer's disease. Neurobiol Aging 27, 596-605.

Dhillon VS, Thomas P, Fenech M (2004) Comparison of DNA damage and repair following radiation challenge in buccal cells and lymphocytes using single-cell gel electrophoresis. Int J Radiat Biol 80, 517-28.

Carlin V, Matsumoto MA, Saraiva PP, Artioli A, Oshima CT, Ribeiro DA (2011) Cytogenetic damage induced by mouthrinses formulations in vivo and in vitro. Clin Oral Investig.

Thomas P, Fenech M (2008) Chromosome 17 and 21 aneuploidy in buccal cells is increased with ageing and in Alzheimer's disease. Mutagenesis 23, 57-65.

Lechel A, Satyanarayana A, Ju Z (2005) The cellular level of telomere dysfunction determines induction of senescence or apoptosis in vivo. EMBO. Rep 6, 275-81.

Herbig U, Sedivy JM (2006) Regulation of growth arrest in senescence: telomere damage is not the end of the story. Mech Ageing Dev 127, 16-24.

de Lange T. (2005) Shelterin: the protein complex that shapes and safeguards human telomeres. Genes Dev 19, 2100-10.

van Steensel B, Smogorzewska A, de Lange T (1998) TRF2 protects human telomeres from end-to-end fusions. Cell 92, 401-13.

Smogorzewska A, de Lange T (2002) Different telomere damage signaling pathways in human and mouse cells. EMBO J 21, 4338-48.

Umen JG (2005) The elusive sizer. Curr Oppinion in Cell Biology 17, 435-441

Echave P, Conlon AC, Lioyd C (2007) Cell size regulation in mammalian cells. Cell Cycle. 6, 218-224.

Broers JL, Ramaekers FC, Bonne G, Yaou RB, Hutchison CJ (2006) Nuclear lamins: laminopathies and their role in premature ageing. Physiol Rev 86, 967-1008.

Taddei A, Hediger F, Neumann FR, Gasser SM (2004) The function of nuclear architecture: a genetic approach. Annu. Rev Genet 38, 305-45.

Dechat T, Pfleghaar K, Sengupta K, Shimi T, Shumaker DK, Solimando L, Goldman RD (2008) Nuclear lamins:major factors in the structural organization and function of the nucleus and chromatin. Genes Dev 22, 832-53.

Mounkes LC, Stewart CL (2004). Aging and nuclear organization: lamins and progeria. Curr Opin Cell Biol 16, 322-327.

Huang S, Risques RA, Martin GM, Rabinovitch PS, Oshima J (2008) Accelerated telomere shortening and replicative senescence in human fibroblasts overexpressing mutant and wild-type lamin A. Exp Cell Res 314, 82-91.

Prokocimer M, Davidovich M, Nissim-Rafinia M, Wiesel-Motiuk N, Bar D, Barkan R, Meshorer E, Gruenbaum Y (2009) Nuclear lamins: key regulators of nuclear structure and activities. J Cell Mol Med 13, 1059-1085.

Raz V, Vermolen BJ, Garini Y, Onderwater JJ, Mommaas-Kienhuis MA, Koster AJ, Young IT, Tanke H, Dirks RW (2008) The nuclear lamina promotes telomere aggregation and centromere peripheral localization during senescence of human mesenchymal stem cells. J Cell Sci 121, 4018-4028.

Gonzalez-Suarez I, Redwood AB, Perkins SM, Vermolen B, Lichtensztejin D, Grotsky DA, Morgado- Palcin L, Gapud EJ, Sleckman BP, Sulivan T, Sage J, Steward CL, Mai S, Gonzalo S (2009) Novel roles for A type lamins in telomere biology and DNA damage response pathway EMBO J 28, 2414-27.

Mai S, Garini Y (2005) Oncogenic remodeling of the three-dimensional organization of the interphase nucleus: c-Myc induces telomeric aggregates whose formation precedes chromosomal rearrangements. Cell Cycle 4, 1327-31.

Gadji M, Fortin D, Tsanacils AM (2010) Three- dimentional Nuclear Telomere architecture is associated with differential time to progression and overall survival in glioblastoma patients. Neoplasia 12, 183-191.

Louis SF, Vermolen BJ, Garini Y, Mai S (2005) c-Myc induces chromosomal rearrangements through telomere and chromosome remodeling in the interphase nucleus. Proc Natl Acad Sci U S A 102, 9613-8.

Guffei A, Sarkar R, Klewes L, Righolt C, Knecht H, Mai S (2010) Dynamic chromosomal rearrangements in Hodg'in's lymphoma are due to ongoing three-dimensional nuclear remodeling and breakage-bridge-fusion cycles. Haematologica 95, 2038-46.

**Claims**

1. A method for evaluating cells derived from a subject suspected of having or having Alzheimer's disease or dementia comprising:

   a) the use of a test cell sample from the subject, the test cell sample comprising buccal cells,
   b) assaying the test cell sample to determine the telomeres organization signature of the test cell sample using three-dimensional (3D) analysis and quantitative fluorescence in situ hybridization (q-FISH), wherein determining the telomeres organization signature comprises detecting one or more of telomere numbers, telomere length and nuclear volume,
   c) comparing the test cell sample telomeres organization signature to one or more control telomeres organization reference signature, and
   d) identifying differences or similarities between the test cell sample telomeres organization signature and the one or more telomeres organization reference signature;

   wherein an increase in the telomere numbers, a decrease in the telomere length and/or a decrease in the nuclear volume in the test cell sample telomeres organization signature compared to the reference telomeres organization signature is indicative the subject has Alzheimer's disease or dementia or an increased risk of developing Alzheimer's disease or dementia.

2. The method of claim 1, wherein the determining the telomeres organization signature comprises detecting telomere length and nuclear volume.

3. The method of claim 1 or 2, wherein determining the test cell sample telomeres organization signature comprises detecting telomere number and a telomere number greater than 60, greater than 70, greater than 80, or greater than 90 is indicative of Alzheimer's disease or dementia or an increased likelihood of developing Alzheimer's disease or dementia.

4. The method of any one of claims 1 to 3, wherein determining the telomeres organization signature comprises detecting nuclear volume and a decrease in the nuclear volume in the test sample telomeres organization signature compared to the reference telomeres organization signature is indicative the subject has Alzheimer's disease and/or dementia or an increased risk of developing Alzheimer's disease and/or dementia.

5. The method of claim 4, wherein a decrease of at least 10, 20, 30, 40 or 50% in the nuclear volume in the test cell sample telomeres organization signature compared to the reference telomeres organization signature is indicative the subject has Alzheimer's disease or dementia or an increased risk of developing Alzheimer's disease or dementia.

6. The method of any one of claims 1 to 5, wherein determining the telomeres organization signature comprises detecting telomeres with a relative fluorescent intensity of (a) less than 20000 units, (b) 20001-40000 units and (c) greater than 40001 units.

7. The method of any one of claims 1 to 6, wherein the Alzheimer's disease is mild Alzheimer's disease, moderate Alzheimer's disease or severe Alzheimer's disease.

8. The method of any one of claims 1 to 7, wherein the telomeres organization signature is determined on interphase telomeres.

9. A method for evaluating cells derived from a subject suspected of having or having Alzheimer's disease or dementia comprising:

   a) using a first test cell sample from the subject, the first test cell sample comprising buccal cells,
   b) using a second test cell sample from the subject, the second test cell sample obtained subsequently to the first test cell sample comprising buccal cells,
   c) assaying the first and second test cell samples to determine the telomeres organization signature of the first and second test cell samples using 3D analysis and q-FISH, wherein determining the telomeres organization signature comprises detecting one or more of telomere numbers, telomere length and nuclear volume,
   d) comparing the first test cell sample telomeres organization signature to the second test cell sample telomeres organization signature, and

e) identifying differences or similarities between the first test cell sample telomeres organization signature and the second test cell sample telomeres organization signature;

wherein a difference in the telomeres organization signature of the second test cell sample compared to the telomeres organization signature of the first test cell sample is indicative the subject has progressing Alzheimer's disease or dementia or ameliorating Alzheimer's disease or dementia and a lack of difference in the telomeres organization signature of the second test cell sample compared to the telomeres organization signature of the first test cell sample is indicative of stable Alzheimer's disease or dementia, the difference being an increase in the telomere numbers, a decrease in the telomere length and/or a decrease in the nuclear volume.

10. The method of claim 9, wherein
the second test cell sample is obtained from the subject after the subject has received one or more treatments, and wherein a difference in the telomeres organization of the second test cell sample compared to the telomeres organization of the first test cell sample is indicative the subject is responding or not responding to the treatment.

11. The method of claim 9 or 10, wherein the determining the telomeres organization signature comprises detecting telomere numbers.

12. The method of any one of claims 1, 9 or 10, wherein the determining the telomeres organization signature comprises detecting telomere length.

13. The method of any one of claims 1 to 12, wherein the determining the telomeres organization signature comprises detecting telomere numbers and telomere length.


**Patentansprüche**

1. Verfahren zur Bewertung von Zellen, die von einem Subjekt stammen, bei dem der Verdacht besteht, dass es an Morbus Alzheimer oder Demenz leidet, oder das an Morbus Alzheimer oder Demenz leidet, umfassend:

a) die Verwendung einer Testzellprobe des Subjekts, wobei die Testzellprobe bukkale Zellen umfasst,
b) Untersuchen der Testzellprobe zur Bestimmung der Telomer-Organisationssignatur der Testzellprobe unter Verwendung von dreidimensionaler (3D) Analyse und quantitativer Fluoreszenz-in situ-Hybridisierung (q-FISH), wobei das Bestimmen der Telomer-Organisationssignatur das Ermitteln eines oder mehrerer der Aspekte Telomerzahl, Telomerlänge und Kernvolumen umfasst,
c) Vergleichen der Telomer-Organisationssignatur der Testzellprobe mit einer oder mehreren Kontroll-Telomer-Organisations-Referenzsignaturen und
d) Identifizieren von Unterschieden oder Ähnlichkeiten zwischen der Telomer-Organisationssignatur der Testzellprobe und der einen oder den mehreren Telomer-Organisations-Referenzsignaturen,

wobei eine Zunahme der Telomerzahl, eine Abnahme der Telomerlänge und/oder eine Abnahme des Kernvolumens in der Telomer-Organisationssignatur der Testzellprobe im Vergleich zur Referenz-Telomer-Organisationssignatur darauf hinweist, dass das Subjekt Morbus Alzheimer oder Demenz hat oder ein erhöhtes Risiko hat, Morbus Alzheimer oder Demenz zu entwickeln.

2. Verfahren nach Anspruch 1, wobei das Bestimmen der Telomer-Organisationssignatur die Ermittlung der Telomerlänge und des Kernvolumens umfasst.

3. Verfahren nach Anspruch 1 oder 2, wobei die Bestimmung der Telomer-Organisationssignatur der Testzellprobe die Ermittlung der Telomerzahl umfasst und eine Telomerzahl von mehr als 60, mehr als 70, mehr als 80 oder mehr als 90 ein Anzeichen für Morbus Alzheimer oder Demenz oder eine erhöhte Wahrscheinlichkeit, Morbus Alzheimer oder Demenz zu entwickeln, ist.

4. Verfahren nach einem der Ansprüche 1 bis 3, wobei das Bestimmen der Telomer-Organisationssignatur die Ermittlung des Kernvolumens umfasst und eine Abnahme des Kernvolumens in der Telomer-Organisationssignatur der Testprobe im Vergleich zur Referenz-Telomer-Organisationssignatur darauf hinweist, dass das Subjekt Morbus Alzheimer und/oder Demenz oder ein erhöhtes Risiko hat, Morbus Alzheimer und/oder Demenz zu entwickeln.

**5.** Verfahren nach Anspruch 4, wobei eine Abnahme von mindestens 10, 20, 30, 40 oder 50 % des Kernvolumens in der Telomer-Organisationssignatur der Testzellprobe im Vergleich zur Referenz-Telomer-Organisationssignatur darauf hinweist, dass das Subjekt Morbus Alzheimer oder Demenz hat oder ein erhöhtes Risiko hat, Morbus Alzheimer oder Demenz zu entwickeln.

**6.** Verfahren nach einem der Ansprüche 1 bis 5, wobei das Bestimmen der Telomer-Organisationssignatur den Nachweis von Telomeren mit einer relativen Fluoreszenzintensität von (a) weniger als 20000 Einheiten, (b) 20001-40000 Einheiten und (c) mehr als 40001 Einheiten umfasst.

**7.** Verfahren nach einem der Ansprüche 1 bis 6, wobei der Morbus Alzheimer ein leichter Morbus Alzheimer, ein mittelschwerer Morbus Alzheimer oder ein schwerer Morbus Alzheimer ist.

**8.** Verfahren nach einem der Ansprüche 1 bis 7, wobei die Telomer-Organisationssignatur an Interphasen-Telomeren bestimmt wird.

**9.** Verfahren zur Bewertung von Zellen, die von einem Subjekt stammen, bei dem der Verdacht besteht, dass es an Morbus Alzheimer oder Demenz leidet, oder das an Morbus Alzheimer oder Demenz leidet, umfassend:

a) Verwenden einer ersten Testzellprobe des Subjekts, wobei die erste Testzellprobe bukkale Zellen umfasst,
b) Verwenden einer zweiten Testzellprobe des Subjekts, wobei die zweite Testzellprobe nach der ersten, bukkale Zellen umfassenden Testzellprobe gewonnen wurde,
c) Untersuchen der ersten und zweiten Testzellproben zur Bestimmung der Telomer-Organisationssignatur der ersten und zweiten Testzellproben unter Verwendung von 3D-Analyse und q-FISH, wobei das Bestimmen der Telomer-Organisationssignatur das Ermitteln eines oder mehrerer der Aspekte Telomerzahl, Telomerlänge und Kernvolumen umfasst,
d) Vergleichen der Telomer-Organisationssignatur der ersten Testzellprobe mit der Telomer-Organisationssignatur der zweiten Testzellprobe und
e) Identifizieren von Unterschieden oder Ähnlichkeiten zwischen der Telomer-Organisationssignatur der ersten Testzellprobe und der Telomer-Organisationssignatur der zweiten Testzellprobe,

wobei ein Unterschied in der Telomer-Organisationssignatur der zweiten Testzellprobe im Vergleich zu der Telomer-Organisationssignatur der ersten Testzellprobe anzeigt, dass das Subjekt sich verschlimmernden Morbus Alzheimer oder Demenz hat oder sich verbessernden Morbus Alzheimer oder Demenz hat, und ein Fehlen eines Unterschieds in der Telomer-Organisationssignatur der zweiten Testzellprobe im Vergleich zu der Telomer-Organisationssignatur der ersten Testzellprobe anzeigt, dass das Subjekt stabilen Morbus Alzheimer oder Demenz hat, wobei der Unterschied eine Zunahme der Telomerzahlen, eine Abnahme der Telomerlänge und/oder eine Abnahme des Kernvolumens ist.

**10.** Verfahren nach Anspruch 9, wobei
die zweite Testzellprobe von dem Subjekt gewonnen wird, nachdem das Subjekt eine oder mehrere Behandlungen erhalten hat, und
wobei ein Unterschied in der Telomerorganisation der zweiten Testzellprobe im Vergleich zur Telomerorganisation der ersten Testzellprobe darauf hinweist, dass das Subjekt auf die Behandlung anspricht oder nicht anspricht.

**11.** Verfahren nach Anspruch 9 oder 10, wobei das Bestimmen der Telomer-Organisationssignatur das Ermitteln der Telomerzahl umfasst.

**12.** Verfahren nach einem der Ansprüche 1, 9 oder 10, wobei das Bestimmen der Telomer-Organisationssignatur das Ermitteln der Telomerlänge umfasst.

**13.** Verfahren nach einem der Ansprüche 1 bis 12, wobei das Bestimmen der Telomer-Organisationssignatur das Ermitteln der Telomerzahl und der Telomerlänge umfasst.

**Revendications**

**1.** Méthode d'évaluation de cellules provenant d'un sujet suspecté d'être atteint ou atteint de la maladie d'Alzheimer ou de démence, comprenant :

a) l'utilisation d'un échantillon cellulaire test provenant du sujet, l'échantillon cellulaire test comprenant des cellules buccales ;

b) le dosage de l'échantillon cellulaire test pour déterminer la signature de l'organisation des télomères de l'échantillon cellulaire test à l'aide d'une analyse tridimensionnelle (3D) et d'une hybridation in situ par fluorescence quantitative (q-FISH), la détermination de la signature de l'organisation des télomères comprenant la détection d'un ou plusieurs éléments parmi le nombre de télomères, la longueur des télomères et le volume nucléaire ;

c) la comparaison de la signature de l'organisation des télomères de l'échantillon cellulaire test à une ou plusieurs signatures de référence de l'organisation des télomères témoins ; et

d) l'identification de différences ou de similitudes entre la signature de l'organisation des télomères de l'échantillon cellulaire test et lesdites une ou plusieurs signatures de référence de l'organisation des télomères ;

une augmentation du nombre de télomères, une diminution de la longueur des télomères et/ou une diminution du volume nucléaire dans la signature de l'organisation des télomères de l'échantillon cellulaire test par rapport à la signature de l'organisation des télomères de référence indique que le sujet est atteint de la maladie d'Alzheimer ou de la démence ou une augmentation du risque de développer la maladie d'Alzheimer ou la démence.

2. Méthode selon la revendication 1, dans laquelle la détermination de la signature d'organisation des télomères comprend la détection de la longueur des télomères et du volume nucléaire.

3. Méthode selon la revendication 1 ou 2, dans laquelle la détermination de la signature de l'organisation des télomères de l'échantillon cellulaire test comprend la détection du nombre de télomères et qu'un nombre de télomères supérieur à 60, supérieur à 70, supérieur à 80 ou supérieur à 90 indique la maladie d'Alzheimer ou la démence ou une probabilité accrue de développer la maladie d'Alzheimer ou la démence.

4. Méthode selon l'une quelconque des revendications 1 à 3, dans laquelle la détermination de la signature de l'organisation des télomères comprend la détection du volume nucléaire et une diminution du volume nucléaire dans la signature de l'organisation des télomères de l'échantillon test par rapport à la signature de l'organisation des télomères de référence indique que le sujet est atteint de la maladie d'Alzheimer et/ou de démence ou un risque accru de développer la maladie d'Alzheimer et/ou la démence.

5. Méthode selon la revendication 4, dans laquelle une diminution d'au moins 10, 20, 30, 40 ou 50 % du volume nucléaire dans la signature de l'organisation des télomères de l'échantillon cellulaire test par rapport à la signature de l'organisation des télomères de référence indique que le sujet est atteint de la maladie d'Alzheimer ou de démence. ou un risque accru de développer la maladie d'Alzheimer ou la démence.

6. Méthode selon l'une quelconque des revendications 1 à 5, dans laquelle la détermination de la signature de l'organisation des télomères comprend la détection des télomères avec une intensité fluorescente relative de (a) moins de 20000 unités, (b) 20001 à 40000 unités et (c) plus de 40001 unités.

7. Méthode selon l'une quelconque des revendications 1 à 6, dans laquelle la maladie d'Alzheimer est la maladie d'Alzheimer légère, la maladie d'Alzheimer modérée ou la maladie d'Alzheimer sévère.

8. Méthode selon l'une quelconque des revendications 1 à 7, dans laquelle la signature de l'organisation des télomères est déterminée sur des télomères en interphase.

9. Méthode d'évaluation de cellules provenant d'un sujet suspecté d'être atteint ou atteint de la maladie d'Alzheimer ou de démence, comprenant :

a) l'utilisation d'un premier échantillon cellulaire test du sujet, le premier échantillon cellulaire test comprenant des cellules buccales,

b) l'utilisation d'un second échantillon cellulaire test provenant du sujet, le second échantillon cellulaire test obtenu ultérieurement au premier échantillon cellulaire test comprenant des cellules buccales,

c) le dosage des premier et second échantillons cellulaires tests pour déterminer la signature de l'organisation des télomères des premier et second échantillons cellulaires tests à l'aide d'une analyse 3D et d'une q-FISH, la détermination de la signature de l'organisation des télomères comprenant la détection d'un ou plusieurs éléments parmi le nombre de télomères, la longueur des télomères et le volume nucléaire,

d) la comparaison de la première signature de l'organisation des télomères des échantillons cellulaires tests à la seconde signature de l'organisation des télomères des échantillons cellulaires tests, et

e) l'identification de différences ou de similitudes entre la première signature de l'organisation des télomères des échantillons cellulaires tests et la seconde signature de l'organisation des télomères des échantillons cellulaires tests ;

une différence dans la signature de l'organisation des télomères du second échantillon cellulaire test par rapport à la signature de l'organisation des télomères du premier échantillon cellulaire test indiquant que le sujet est atteint d'une maladie d'Alzheimer ou de démence évolutive ou d'une maladie d'Alzheimer ou de démence en amélioration et un manque de différence dans la signature de l'organisation des télomères du second échantillon cellulaire test par rapport à la signature de l'organisation des télomères du premier échantillon cellulaire test indiquant une maladie d'Alzheimer ou une démence stable, la différence étant une augmentation du nombre de télomères, une diminution de la longueur des télomères et/ou une diminution du volume nucléaire.

10. Méthode selon la revendication 9, dans laquelle :

le second échantillon cellulaire test est obtenu du sujet après que celui-ci ait reçu un ou plusieurs traitements, et une différence dans l'organisation des télomères du second échantillon cellulaire test par rapport à l'organisation des télomères du premier échantillon cellulaire test indiquant que le sujet répond ou ne répond pas au traitement.

11. Méthode selon la revendication 9 ou 10, dans laquelle la signature de l'organisation des télomères comprend la détection du nombre de télomères.

12. Méthode selon l'une quelconque des revendications 1, 9 ou 10, dans laquelle la détermination de la signature de l'organisation des télomères comprend la détection de la longueur des télomères.

13. Méthode selon l'une quelconque des revendications 1 à 12, dans laquelle la détermination de la signature de l'organisation des télomères comprend la détection du nombre de télomères et de la longueur des télomères.

**FIGURE 1A**

**FIGURE 1B**

**FIGURE 1C**

FIGURE 2

Control          AD Mild

2D

3D

**FIGURE 3A**

Control          Moderate AD

2D

3D

FIGURE 3B

FIGURE 3C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7801682 B

**Non-patent literature cited in the description**

- **KNECHT H ; SAWAN B ; LICHTENSZTEJN Z ; LICHTENSZTEJN D ; MAI S.** *Lab Invest.,* 2010, vol. 90 (4), 611-619
- **AD. FURTHER ; THOMAS et al.** *teaches that,* 2008
- **BLACKBURN, E. H.** *Nature,* 1991, vol. 350, 569-573
- **GREIDER, C. W.** *Annu Rev Biochem,* 1996, vol. 65, 337-365
- **BLACKBURN, E. H.** *Annu Rev Biochem,* 1992, vol. 61, 113-129
- **BUCHKOVICH, K. J. ; GREIDER, C. W.** *Mol Biol Cell,* 1996, vol. 7, 1443-1454
- **SLAGBOOM, P. E. ; DROOG, S. ; BOOMSMA, D. I.** *Am J Hum Genet,* 1994, vol. 55, 876-882
- **RUDOLPH, K. L. ; CHANG, S. ; LEE, H. W. ; BLASCO, M. ; GOTTLIEB, G. J. ; GREIDER, C. ; DEPINHO, R. A.** *Cell,* 1999, vol. 96, 701-712
- **CHAN, S. W. ; BLACKBURN, E. H.** *Oncogene,* 2002, vol. 21, 553-563
- **FRY, M. ; LOEB, L. A.** *J Biol Chem,* 1999, vol. 274, 12797-12802
- **SHEN, J. C. ; GRAY, M. D. ; OSHIMA, J. ; KAMATH-LOEB, A. S. ; FRY, M. ; LOEB, L. A.** *J Biol Chem,* 1998, vol. 273, 34139-34144
- **YU, C. E. ; OSHIMA, J. ; FU, Y. H. ; WIJSMAN, E. M. ; HISAMA, F. ; ALISCH, R. ; MATTHEWS, S. ; NAKURA, J. ; MIKI, T. ; OUAIS, S.** *Science,* 1996, vol. 272, 258-262
- **HAYFLICK, L.** *Handbook of the bilology aging,* 1977, vol. 20
- **SOHAL, R. S. ; ALLEN, R. G.** *Basic Life Sci,* 1985, vol. 35, 75-104
- **VULLIAMY, T. J. ; KNIGHT, S. W. ; MASON, P. J. ; DOKAL, I.** *Blood Cells Mol Dis,* 2001, vol. 27, 353-357
- **CHARAMES, G. S. ; BAPAT, B.** *Curr Mol Med,* 2003, vol. 3, 589-596
- **HOLLAND, A. J. ; CLEVELAND, D. W.** *Nat Rev Mol Cell Biol,* 2009, vol. 10, 478-487
- **KNECHT, H. ; BRUDERLEIN, S. ; WEGENER, S. ; LICHTENSZTEJN, D. ; LICHTENSZTEJN, Z. ; LEMIEUX, B. ; MOLLER, P. ; MAI, S.** *BMC Cell Biol,* vol. 11, 99
- **LOTHE, R. A. ; PELTOMAKI, P ; MELING, G. I. ; AALTONEN, L. A. ; NYSTROM-LAHTI, M., PYLKKANEN, L. ; HEIMDAL, K. ; ANDERSEN, T. I. ; MOLLER, P. ; ROGNUM, T. O. et al.** *Cancer Res,* 1993, vol. 53, 5849-5852
- **COLLEU-DUREL S, G. N. ; NOURGALIEVA K ; LÉVÊQUE J ; DANIC B ; CHENAL C.** *Oncol Rep,* 2001, vol. 8, 4
- **GUFFEI, A. ; SARKAR, R. ; KLEWES, L. ; RIGHOLT, C. ; KNECHT, H. ; MAI, S.** *Haematologica,* vol. 95, 2038-2046
- **LANGE, T.** *Oncogene,* 2002, vol. 21, 532-540
- **MASUTOMI, K. ; YU, E. Y. ; KHURTS, S. ; BEN-PORATH, I. ; CURRIER, J. L. ; METZ, G. B. ; BROOKS, M. W. ; KANEKO, S. ; MURAKAMI, S. ; DECAPRIO, J. A.** *Cell,* 2003, vol. 114, 241-253
- **HARLEY, C. B. ; FUTCHER, A. B. ; GREIDER, C. W.** *Nature,* 1990, vol. 345, 458-460
- **HUFFMAN, K. E. ; LEVENE, S. D. ; TESMER, V. M. ; SHAY, J. W. ; WRIGHT, W. E.** *J Biol Chem,* 2000, vol. 275, 19719-19722
- **KIRWAN, M. ; DOKAL, I.** *Biochim Biophys Acta,* 2009, vol. 1792, 371-379
- **CHANG, S. ; MULTANI, A. S. ; CABRERA, N. G. ; NAYLOR, M. L. ; LAUD, P. ; LOMBARD, D. ; PATHAK, S. ; GUARENTE, L. ; DEPINHO, R. A.** *Nat Genet,* 2004, vol. 36, 877-882
- **THOMAS, P. ; NJ, O. C. ; FENECH, M.** *Mech Ageing Dev,* 2008, vol. 129, 183-190
- **THOMAS, P. ; HECKER, J. ; FAUNT, J. ; FENECH, M.** *Mutagenesis,* 2007, vol. 22, 371-379
- **PANOSSIAN, L. A. ; PORTER, V. R. ; VALENZUELA, H. F. ; ZHU, X. ; REBACK, E. ; MASTERMAN, D. ; CUMMINGS, J. L. ; EFFROS, R. B.** *Neurobiol Aging,* 2003, vol. 24, 77-84
- **BURNS, A. ; BYRNE, E. J. ; MAURER, K.** *Lancet,* 2002, vol. 360, 163-165
- **DU, A. T. ; SCHUFF, N. ; AMEND, D. ; LAAKSO, M. P. ; HSU, Y. Y. ; JAGUST, W. J. ; YAFFE, K. ; KRAMER, J. H. ; REED, B. ; NORMAN, D.** *J Neurol Neurosurg Psychiatry,* 2001, vol. 71, 441-447

- **HAROUTUNIAN, V. ; PERL, D. P. ; PUROHIT, D. P. ; MARIN, D. ; KHAN, K. ; LANTZ, M. ; DAVIS, K. L. ; MOHS, R. C.** *Arch Neurol,* 1998, vol. 55, 1185-1191
- **KAWAS, C. H.** *N Engl J Med,* 2003, vol. 349, 1056-1063
- **MATTSON, M. P.** *Nature,* 2004, vol. 430, 631-639
- **IQBAL, K. ; GRUNDKE-IQBAL, I. ; SMITH, A. J. ; GEORGE, L. ; TUNG, Y. C. ; ZAIDI, T.** *Proc Natl Acad Sci U S A,* 1989, vol. 86, 5646-5650
- **HUNT, A. J. ; MCINTOSH, J. R.** *Mol Biol Cell,* 1998, vol. 9, 2857-2871
- **IQBAL, K. ; ALONSO, A. C. ; GONG, C. X. ; KHATOON, S. ; PEI, J. J., WANG, J. Z. ; GRUNDKE-IQBAL, I.** *J Neural Transm Suppl,* 1998, vol. 53, 169-180
- **PETKOVA, A. T. ; ISHII, Y. ; BALBACH, J. J. ; ANTZUTKIN, O. N. ; LEAPMAN, R. D. ; DELAGLIO, F. ; TYCKO, R.** *Proc Natl Acad Sci U S A,* 2002, vol. 99, 16742-16747
- **ANTZUTKIN, O. N. ; LEAPMAN, R. D. ; BALBACH, J. J. ; TYCKO, R.** *Biochemistry,* 2002, vol. 41, 15436-15450
- **THOMAS, P. ; FENECH, M.** *Mutagenesis,* 2008, vol. 23, 57-65
- **NASREDDINE, Z. S. ; PHILLIPS, N. A. ; BEDIRIAN, V. ; CHARBONNEAU, S. ; WHITEHEAD, V. ; COLLIN, I. ; CUMMINGS, J. L. ; CHERTKOW, H.** *J Am Geriatr Soc,* 2005, vol. 53, 695-699
- **FOLSTEIN, M. F. ; FOLSTEIN, S. E. ; MCHUGH, P. R.** *J Psychiatr Res,* 1975, vol. 12, 189-198
- **MCKHANN, G. ; DRACHMAN, D. ; FOLSTEIN, M. ; KATZMAN, R. ; PRICE, D. ; STADLAN, E. M.** *Neurology,* 1984, vol. 34, 939-944
- **PATTERSON, C. J. ; GAUTHIER, S. ; BERGMAN, H. ; COHEN, C. A. ; FEIGHTNER, J. W. ; FELDMAN, H. ; HOGAN, D. B.** *CMAJ,* 1999, vol. 160, 1738-1742
- **CHUANG, T. C. ; MOSHIR, S. ; GARINI, Y. ; CHUANG, A. Y. ; YOUNG, I. T. ; VERMOLEN, B. ; VAN DEN DOEL, R. ; MOUGEY, V. ; PERRIN, M. ; BRAUN, M.** *BMC Biol,* 2004, vol. 2, 12
- **LOUIS, S. F. ; VERMOLEN, B. J. ; GARINI, Y. ; YOUNG, I. T. ; GUFFEI, A. ; LICHTENSZTEJN, Z. ; KUTTLER, F. ; CHUANG, T. C. ; MOSHIR, S. ; MOUGEY, V.** *Proc Natl Acad Sci U S A,* 2005, vol. 102, 9613-9618
- **KLEWES, L. ; HÖBSCH, C. ; KATZIR, N. ; ROURKE, D. ; GARINI, Y. ; MAI, S.** *Cytometry Part A,* 2011, vol. 79A (47), 159-166
- **BLACKBURN EH.** Structure and function of telomeres. *Nature,* 1991, vol. 350, 569-73
- **LANGE T.** Protection of mammalian telomeres. *Oncogene,* 2002, vol. 21, 532-40
- **HARLEY CB ; FUTCHER AB ; GREIDER CW.** Telomeres shorten during ageing of human fibroblasts. *Nature,* 1990, vol. 345, 458-60
- **HUFFMAN KE ; LEVENE SD ; TESMER VM ; SHAY JW ; WRIGHT WE.** Telomere shortening is proportional to the size of the G-rich telomeric 3'-overhang. *J Biol Chem,* 2000, vol. 275, 19719-22
- **CHARAMES GS ; BAPAT B.** Genomic instability and cancer. *Curr Mol Med,* 2003, vol. 3, 589-96
- **HOLLAND AJ ; CLEVELAND DW.** Boveri revisited: chromosomal instability, aneuploidy and tumorigenesis. *Nat Rev Mol Cell Biol,* 2009, vol. 10, 478-87
- **LOTHE RA ; PELTOMAKI P ; MELING GI.** Genomic instability in colorectal cancer: relationship to clinicopathological variables and family history. *Cancer Res,* 1993, vol. 53, 5849-52
- **COLLEU-DUREL S ; GUITTON N ; NOURGALIEVA K ; LEVEQUE J ; DANIC B ; CHENAL C.** Genomic instability and breast cancer. *Oncol Rep,* 2001, vol. 8, 1001-5
- **CHAN SW ; BLACKBURN EH.** New ways not to make ends meet: telomerase, DNA damage proteins and heterochromatin. *Oncogene,* 2002, vol. 21, 553-63
- **HAYFLICK, L.** The Cell Biology of Aging. *Journal of Investigative Dermatology,* 1979, vol. 73, 8-14
- **SOHAL RS ; ALLEN RG.** Relationship between metabolic rate, free radicals, differentiation and aging: a unified theory. *Basic Life Sci,* 1985, vol. 35, 75-104
- **YU CE ; OSHIMA J ; FU YH.** Positional cloning of the Werner's syndrome gene. *Science,* 1996, vol. 272, 258-62
- **SHEN JC ; GRAY MD ; OSHIMA J ; KAMATH-LOEB AS ; FRY M ; LOEB LA.** Werner syndrome protein. I. DNA helicase and dna exonuclease reside on the same polypeptide. *J Biol Chem,* 1998, vol. 273, 34139-44
- **FRY M ; LOEB LA.** Human werner syndrome DNA helicase unwinds tetrahelical structures of the fragile X syndrome repeat sequence d(CGG)n. *J Biol Chem,* 1999, vol. 274, 12797-802
- **BURNS A ; BYRNE EJ ; MAURER K.** Alzheimer's disease. *Lancet,* 2002, vol. 360, 163-5
- **PANOSSIAN LA ; PORTER VR ; VALENZUELA HF.** Telomere shortening in T cells correlates with Alzheimer's disease status. *Neurobiol Aging,* 2003, vol. 24, 77-84
- **THOMAS P ; HECKER J ; FAUNT J ; FENECH M.** Buccal micronucleus cytome biomarkers may be associated with Alzheimer's disease. *Mutagenesis,* 2007, vol. 22, 371-9
- **DU AT ; SCHUFF N ; AMEND D.** Magnetic resonance imaging of the entorhinal cortex and hippocampus in mild cognitive impairment and Alzheimer's disease. *J Neurol Neurosurg Psychiatry,* 2001, vol. 71, 441-7

- **HAROUTUNIAN V ; PERL DP ; PUROHIT DP.** Regional distribution of neuritic plaques in the nondemented elderly and subjects with very mild Alzheimer disease. *Arch Neurol,* 1998, vol. 55, 1185-91
- **MATTSON MP.** Pathways towards and away from Alzheimer's disease. *Nature,* 2004, vol. 430, 631-9
- **KAWAS CH.** Clinical practice. Early Alzheimer's disease. *N Engl J Med,* 2003, vol. 349, 1056-63
- **IQBAL K ; ALONSO AC ; GONG CX.** Mechanisms of neurofibrillary degeneration and the formation of neurofibrillary tangles. *J Neural Transm Suppl,* 1998, vol. 53, 169-80
- **PETKOVA AT ; ISHII Y ; BALBACH JJ.** A structural model for Alzheimer's beta - amyloid fibrils based on experimental constraints from solid state NMR. *Proc Natl Acad Sci U S A,* 2002, vol. 99, 16742-7
- **ANTZUTKIN ON ; LEAPMAN RD ; BALBACH JJ ; TYCKO R.** Supramolecular structural constraints on Alzheimer's beta-amyloid fibrils from electron microscopy and solid-state nuclear magnetic resonance. *Biochemistry,* 2002, vol. 41, 15436-50
- **THOMAS P ; NJ OC ; FENECH M.** Telomere length in white blood cells, buccal cells and brain tissue and its variation with ageing and Alzheimer's disease. *Mech Ageing Dev,* 2008, vol. 129, 183-90
- **CAWTHON RM ; SMITH KR ; O'BRIEN E ; SIVATCHENKO A ; KERBER RA.** Association between telomere length in blood and mortality in people aged 60 years or older. *Lancet,* 2003, vol. 361, 393-5
- **HONIG SL ; TANG MX ; ALBERT S ; COSTA R.** Stroke and the Risk of Alzheimer Disease. *Archives of Neurology,* 2003, vol. 60, 1707-1712
- **LUKENS JN ; VAN DEERLIN V ; CLARK CM ; XIE SX ; JOHNSON FB.** Comparisons of telomere lengths in peripheral blood and cerebellum in Alzheimer's disease. *Alzheimers Dement,* 2009, vol. 5, 463-9
- **VERMOLEN BJ ; GARINI Y ; MAI S.** Characterizing the three-dimensional organization of telomeres. *Cytometry A,* 2005, vol. 67, 144-50
- **MCKHANN G ; DRACHMAN D ; FOLSTEIN M ; KATZMAN R ; PRICE D ; STADLAN EM.** Clinical diagnosis of Alzheimer's disease: report of the NINCDS-ADRDA Work Group under the auspices of Department of Health and Human Services Task Force on Alzheimer's Disease. *Neurology,* 1984, vol. 34, 939-44
- **HOGAN DB ; BAILEY P ; BLACK S.** *Diagnosis and treatment of dementia,* 2008, vol. 5
- Nonpharmacologic and pharmacologic therapy for mild to moderate dementia. *CMAJ,* vol. 176, 1019-1026
- **NASREDDINE ZS ; PHILLIPS NA ; BEDIRIAN V.** The Montreal Cognitive Assessment, MoCA: a brief screening tool for mild cognitive impairment. *J Am Geriatr Soc,* 2005, vol. 53, 695-9
- **FOLSTEIN MF ; FOLSTEIN SE ; MCHUGH PR.** Mini-mental state". A practical method for grading the cognitive state of patients for the clinician. *J Psychiatr Res,* 1975, vol. 12, 189-98
- **KNECHT H ; SAWAN B ; LICHTENSZTEJN D ; LEMIEUX B ; WELLINGER RJ ; MAI S.** The 3D nuclear organization of telomeres marks the transition from Hodgkin to Reed-Sternberg cells. *Leukemia,* 2009, vol. 23, 565-73
- **KNECHT H ; BRUDERLEIN S ; WEGENER S ; MAI S.** 3D nuclear organization of telomeres in the Hodgkin cell lines U-HO1 and U-HO1-PTPN1: PTPN1 expression prevents the formation of very short telomeres including "t-stumps. *BMC Cell Biol,* 2010, vol. 11, 99-102
- **SCHAEFER LH ; SCHUSTER D ; HERZ H.** Generalized approach for accelerated maximum likelihood based image restoration applied to three-dimensional fluorescence microscopy. *J Microsc,* 2010, vol. 204, 99-107
- **POON SS ; MARTENS UM ; WARD RK ; LANSDORP PM.** Telomere length measurements using digital fluorescence microscopy. *Cytometry,* 1999, vol. 36, 267-78
- **MAI S ; GARINI Y.** The significance of telomeric aggregates in the interphase nuclei of tumor cells. *J Cell Biochem,* 2006, vol. 97, 904-15
- **MILYAVSKY M ; MIMRAN A ; SENDEROVICH S.** Activation of p53 protein by telomeric (TTAGGG)n repeats. *Nucleic Acids Res,* 2001, vol. 29, 5207-15
- **FARAZI PA ; GLICKMAN J ; HORNER J ; DEPINHO RA.** Cooperative interactions of p53 mutation, telomere dysfunction, and chronic liver damage in hepatocellular carcinoma progression. *Cancer Res,* 2006, vol. 66, 4766-73
- **MYUNG NH ; ZHU X ; KRUMAN, II.** Evidence of DNA damage in Alzheimer disease: phosphorylation of histone H2AX in astrocytes. *Age (Dordr),* 2008, vol. 30, 209-15
- **COPPEDE F ; MIGLIORE L.** DNA damage and repair in Alzheimer's disease. *Curr Alzheimer Res,* 2009, vol. 6, 36-47
- **MIRZOEVA OK ; PETRINI JH.** DNA damage-dependent nuclear dynamics of the Mre11 complex. *Mol Cell Biol,* 2001, vol. 21, 281-8
- **DELMAS S ; SHUNBURNE L ; NGO HP ; ALLERS T.** Mre11-Rad50 promotes rapid repair of DNA damage in the polyploid archaeon Haloferax volcanii by restraining homologous recombination. *PLoS Genet,* 2009, vol. 5, e1000552
- **JACOBSON SJ ; LAURENSON PM ; PILLUS L.** Functional analyses of chromatin modifications in yeast. *Methods Enzymol,* 2004, vol. 377, 3-55
- **DAVYDOV V ; HANSEN LA ; SHACKELFORD DA.** Is DNA repair compromised in Alzheimer's disease. *Neurobiol Aging,* 2003, vol. 24, 953-68

- **DAVYDOV V ; HANSEN LA ; SHACKELFORD DA.** s DNA repair compromised in Alzheimer's disease. *Neurobiol Aging,* 2003, vol. 24, 953-68
- **SHACKELFORD DA.** DNA end joining activity is reduced in Alzheimer's disease. *Neurobiol Aging,* 2006, vol. 27, 596-605
- **DHILLON VS ; THOMAS P ; FENECH M.** Comparison of DNA damage and repair following radiation challenge in buccal cells and lymphocytes using single-cell gel electrophoresis. *Int J Radiat Biol,* 2004, vol. 80, 517-28
- **CARLIN V ; MATSUMOTO MA ; SARAIVA PP ; ARTIOLI A ; OSHIMA CT ; RIBEIRO DA.** Cytogenetic damage induced by mouthrinses formulations in vivo and in vitro. *Clin Oral Investig,* 2011
- **THOMAS P ; FENECH M.** Chromosome 17 and 21 aneuploidy in buccal cells is increased with ageing and in Alzheimer's disease. *Mutagenesis,* 2008, vol. 23, 57-65
- **LECHEL A ; SATYANARAYANA A ; JU Z.** The cellular level of telomere dysfunction determines induction of senescence or apoptosis in vivo. *EMBO. Rep,* 2005, vol. 6, 275-81
- **HERBIG U ; SEDIVY JM.** Regulation of growth arrest in senescence: telomere damage is not the end of the story. *Mech Ageing Dev,* 2006, vol. 127, 16-24
- **LANGE T.** Shelterin: the protein complex that shapes and safeguards human telomeres. *Genes Dev,* 2005, vol. 19, 2100-10
- **VAN STEENSEL B ; SMOGORZEWSKA A ; LANGE T.** TRF2 protects human telomeres from end-to-end fusions. *Cell,* 1998, vol. 92, 401-13
- **SMOGORZEWSKA A ; LANGE T.** Different telomere damage signaling pathways in human and mouse cells. *EMBO J,* 2002, vol. 21, 4338-48
- **UMEN JG.** The elusive sizer. *Curr Oppinion in Cell Biology,* 2005, vol. 17, 435-441
- **ECHAVE P ; CONLON AC ; LIOYD C.** Cell size regulation in mammalian cells. *Cell Cycle,* 2007, vol. 6, 218-224
- **BROERS JL ; RAMAEKERS FC ; BONNE G ; YAOU RB ; HUTCHISON CJ.** Nuclear lamins: laminopathies and their role in premature ageing. *Physiol Rev,* 2006, vol. 86, 967-1008
- **TADDEI A ; HEDIGER F ; NEUMANN FR ; GASSER SM.** The function of nuclear architecture: a genetic approach.Annu. *Rev Genet,* 2004, vol. 38, 305-45
- **DECHAT T ; PFLEGHAAR K ; SENGUPTA K ; SHIMI T ; SHUMAKER DK ; SOLIMANDO L ; GOLDMAN RD.** Nuclear lamins:major factors in the structural organization and function of the nucleus and chromatin. *Genes Dev,* 2008, vol. 22, 832-53

- **MOUNKES LC ; STEWART CL.** Aging and nuclear organization: lamins and progeria. *Curr Opin Cell Biol,* 2004, vol. 16, 322-327
- **HUANG S ; RISQUES RA ; MARTIN GM ; RABINOVITCH PS ; OSHIMA J.** Accelerated telomere shortening and replicative senescence in human fibroblasts overexpressing mutant and wild-type lamin A. *Exp Cell Res,* 2008, vol. 314, 82-91
- **PROKOCIMER M ; DAVIDOVICH M ; NISSIM-RAFINIA M ; WIESEL-MOTIUK N ; BAR D ; BARKAN R ; MESHORER E ; GRUENBAUM Y.** Nuclear lamins: key regulators of nuclear structure and activities. *J Cell Mol Med,* 2009, vol. 13, 1059-1085
- **RAZ V ; VERMOLEN BJ ; GARINI Y ; ONDERWATER JJ ; MOMMAAS-KIENHUIS MA ; KOSTER AJ ; YOUNG IT ; TANKE H ; DIRKS RW.** The nuclear lamina promotes telomere aggregation and centromere peripheral localization during senescence of human mesenchymal stem cells. *J Cell Sci,* 2008, vol. 121, 4018-4028
- **GONZALEZ-SUAREZ I ; REDWOOD AB ; PERKINS SM ; VERMOLEN B ; LICHTENSZTEJIN D ; GROTSKY DA ; MORGADO- PALCIN L ; GAPUD EJ ; SLECKMAN BP ; SULIVAN T.** Novel roles for A type lamins in telomere biology and DNA damage response pathway. *EMBO J,* 2009, vol. 28, 2414-27
- **MAI S ; GARINI Y.** Oncogenic remodeling of the three-dimensional organization of the interphase nucleus: c-Myc induces telomeric aggregates whose formation precedes chromosomal rearrangements. *Cell Cycle,* 2005, vol. 4, 1327-31
- **GADJI M ; FORTIN D ; TSANACILS AM.** Three- dimentional Nuclear Telomere architecture is associated with differential time to progression and overall survival in glioblastoma patients. *Neoplasia,* 2010, vol. 12, 183-191
- **LOUIS SF ; VERMOLEN BJ ; GARINI Y ; MAI S.** c-Myc induces chromosomal rearrangements through telomere and chromosome remodeling in the interphase nucleus. *Proc Natl Acad Sci U S A,* 2005, vol. 102, 9613-8
- **GUFFEI A ; SARKAR R ; KLEWES L ; RIGHOLT C ; KNECHT H ; MAI S.** Dynamic chromosomal rearrangements in Hodg'in's lymphoma are due to ongoing three-dimensional nuclear remodeling and breakage-bridge-fusion cycles. *Haematologica,* 2010, vol. 95, 2038-46